# EUROPÄISCHE PATENTSCHRIFT

(11) **EP 3 114 105 B1**
(45) Veröffentlichungstag und Bekanntmachung des Hinweises auf die Patenterteilung: **09.05.2018**
(21) Anmeldenummer: 15705590.6
(22) Anmeldetag: 16.02.2015
(51) Int. Cl.: C07C 47/02, C07C 45/50

(54) **VERFAHREN ZUR HERSTELLUNG VON ALDEHYDEN AUS ALKANEN UND SYNTHESEGAS**
METHOD FOR PRODUCING ALDEHYDES FROM ALKANES AND SYNTHESIS GAS
PROCÉDÉ DE PRÉPARATION D'ALDÉHYDES À PARTIR D'ALCANES ET DE GAZ DE SYNTHÈSE

(30) Priorität: 05.03.2014 DE 102014203960
(43) Veröffentlichungstag der Anmeldung: 11.01.2017
(73) Patentinhaber: Evonik Degussa GmbH, 45128 Essen (DE)
(72) Erfinder: FRANKE, Robert, 45772 Marl (DE); HAHN, Hanna, 47199 Duisburg-Baerl (DE); QUANDT, Thomas, 45772 Marl (DE); WASSERSCHEID, Peter, 91054 Erlangen (DE); HAUMANN, Marco, 91074 Herzogenaurach (DE); WALTER, Simon, 79595 Rümmingen (DE)
(86) Internationale Anmeldenummer: PCT/EP2015/053180
(87) Internationale Veröffentlichungsnummer: WO 2015/132068

(56) Entgegenhaltungen:
- WO-A1-2004/041763
- WO-A1-2011/157788
- US-A1- 2004 024 259
- US-A1- 2013 289 313

## Beschreibung

Gegenstand der vorliegenden Erfindung ist ein Verfahren zur Herstellung von einem oder mehreren Aldehyden aus einem oder mehreren Alkanen, umfassend die Schritte: a) Dehydrieren mindestens eines Alkans in Gegenwart eines heterogenen Katalysators zu mindestens einem Alken, b) Umsetzen des Alkens oder der Alkene aus Schritt a) mit Wasserstoff und Kohlenmonoxid in Gegenwart eines heterogenen Katalysators, der von dem Katalysator in Schritt a) verschieden ist, welches dadurch gekennzeichnet ist, dass als Katalysator in Schritt b) eine katalytisch wirksame Zusammensetzung eingesetzt wird, die ein festes Trägermaterial aufweist, welches eine ionische Flüssigkeit trägt, in welcher mindestens ein Übergangsmetall und mindestens ein Ligand aufweisender Katalysator enthalten ist, sowie eine Apparatur, geeignet zur Dehydrierung von einem oder mehreren Alkanen zu einem oder mehreren Alkenen und Hydroformylierung des erhaltenen Alkens bzw. der erhaltenen Alkene zu Aldehyden, welche dadurch gekennzeichnet ist, dass sie mindestens zwei Reaktionszonen aufweist, die fluidleitend miteinander verbunden sind, wobei eine erste Reaktionszone als Festbett ausgeführt ist und einen Katalysator aufweist, der die Dehydrierung von Alkanen zu Alkenen katalysiert und eine zweite Reaktionszone als Festbett ausgeführt ist, die einen Katalysator aufweist, der die Hydroformylierung von Alkenen katalysiert und der ein festes Trägermaterial aufweist, welches eine ionische Flüssigkeit trägt, in welcher mindestens ein Übergangsmetall und mindestens ein Ligand enthalten ist.

In der organischen Chemie werden Stoffgruppen gemeinhin nach der Anzahl ihrer Kohlenstoffatome eingeteilt. Dabei wird der interessierenden Stoffklasse das Präfix Cₙ-vorangestellt, wobei n für die Anzahl der jeweiligen Kohlenstoffatome des Stoffes steht. Ist beispielsweise von C₄-Alkenen die Rede, so sind die vier isomeren Olefine mit vier Kohlenstoffatomen gemeint, nämlich Isobuten, 1-Buten, cis-2-Buten und trans-2-Buten. Demgegenüber existiert nur ein Alken mit drei Kohlenstoffatomen, nämlich Propen und ein C₃-Alkan, nämlich Propan.

Das erfindungsgemäße Verfahren kann insbesondere dann vorteilhaft eingesetzt werden, wenn es sich bei dem Alkan um Cₙ-Alkane (n=3, 4 oder 5) oder deren Mischungen handelt, bei dem Alken um Cₙ-Alkene (n= 3, 4, 5) und deren Mischungen und bei dem Aldehyd um Cₙ₊₁-Aldehyde und deren Mischungen handelt.

Die katalytische Dehydrierung von C₁-C₁₀ Alkanen ist heutzutage eine wichtige industrielle Route um selektiv Olefine herzustellen. Speziell die überproportional steigende Nachfrage nach Propen und Buten gegenüber Ethen führte zu einer Intensivierung dieser Technologie. Ein Großteil von Propen und Buten wird nach wie vor durch das Steamcracking und Fluid Catalytic Cracking (FCC) gewonnen. Die Selektivität zu einem bestimmten Produkt kann nur bedingt durch veränderte Prozessbedingungen beim Steamcracking und FCC gesteigert werden. Die Produktverteilung und damit einhergehend die Trägheit auf eine schnellere Nachfrage am Markt zu reagieren, sorgen für ein konstantes Interesse in die Technologie der katalytische Dehydrierung von Alkanen.

Katalytische Dehydrierung von Alkanen wird bereits seit den 1930 Jahren durchgeführt. Leuna in Deutschland und UOP (Universal Oil Products) in den USA zusammen mit ICI in England haben unabhängig voneinander die Technik kommerzialisiert. Beide Verfahren verwendeten einen Chrom/Aluminiumoxid Katalysator. Bis heute sind Chromoxid auf Aluminiumoxid und Platin/Zinn auf Aluminiumoxid die industriell am meisten verwendeten Katalysatoren zur katalytischen Dehydrierung von C₁-C₁₀ Alkanen. Beide Metalle unterscheiden sich nicht wesentlich in der Aktivität, aber in der Selektivität, Stabilität, Regenerierbarkeit und Preis. (D. Sanfilippo, I. Miracca, 'Dehydrogenation of paraffins: synergies between catalyst design and reactor engineering', *Catal. Today* **2006,** *111*, 133-139.) Ein Vorteil von PtSn-Katalysatoren ist, dass sie fähig zur Strukturisomerie (i-C₄ ↔ n-C₄, i bezeichnet das verzweigte Isomer, n das unverzweigte Isomer) sein können. Sowohl Chrom- als auch Platin-Katalysatoren haben gemeinsam, dass sie aufgrund von Koksbildung deaktivieren. Chrom-Katalysatoren sind zwischen einigen Minuten und einigen Stunden aktiv, Platin-Katalysatoren einige Stunden bis Tage. Folglich müssen die Katalysatoren periodisch regeneriert werden. Die Regeneration erfolgt meistens mit Luft (und/oder Dampf), um die Kohlenstoffablagerungen abzubrennen. Durch die hydrothermalen Bedingungen sollten die Katalysatoren nicht verändert werden. Platin zum Beispiel neigt zum Sintern bei hohen Temperaturen während der Regeneration. In manchen Fällen ist eine Reduzierung des Metalls vor dem Kontakt mit Kohlenwasserstoffen oder eine Restrukturierung des Metalls mit Chloriden nötig. Die Reduktion kann auch in-situ durch frei werdenden Wasserstoff während der Dehydrierung stattfinden. Es existieren zahlreiche kommerzielle Verfahren, die sich hinsichtlich Temperaturmanagement, Katalysatorführung und Katalysatorregenerierung unterscheiden.

Das CATOFIN Verfahren setzt auf parallel angeordnete Festbettreaktoren. Es beruht auf dem von Houdry entwickelten CATADIENE Prozess zur Produktion von Butadien (GB 778821(**1957**), Houdry Process Corp.; R. M. Shirk, R. G. Carig, A. W. Hoge, GB 794089 (1958), Houdry Process Corp.).

Das OLEFLEX Verfahren besteht aus in Reihe geschalteten Rohrreaktoren, die adiabat betrieben werden. Der Platin/Zinn auf Aluminiumoxid-Katalysator passiert parallel mit dem Reaktionsgas die Reaktoren. Der Katalysator wird in einer Regenerationseinheit aufgefangen und kontinuierlich dem Prozess wieder zugeführt. Das Reaktionsgas wird vor dem Eintritt und zwischen den Reaktoren durch Gasbrenner auf Reaktionstemperatur erhitzt.

In dem STAR-Verfahren von Thyssen-Krupp Uhde werden die Festbettreaktoren aktiv mit Brenner analog dem Steam-Reforming beheizt. Das Verfahren beruht auf der oxidative Dehydrierung. Die Reaktoren werden normalerweise mit einem Druck von 0,5 MPa betrieben. Der Partialdruck der Kohlenwasserstoffe wird mit Dampf und/oder Luft reduziert. Der erhöhte Druck ist prozessbedingt und wirkt sich positiv auf die anschließende Aufreinigung und folglich die Energiebilanz der Anlage aus. Reaktion und Regeneration erfolgen in einem Zyklus, daher werden meist mehrere Reaktoren parallel eingesetzt. Der Katalysator ist ein Platin/Zinn auf Magnesium/Zink dotiertem Aluminat (D. Sanfilippo, P. N. Rylander, in Ullmann's Encyclopedia of Industrial Chemistry, Wiley-VCH Verlag GmbH & Co. KGaA, 2000.).

Das Reaktorkonzept des PDH Verfahrens besteht aus parallel angeordneten Festbettreaktoren, die mit Brenner befeuert werden. Die Regeneration des Katalysators erfolgt zyklisch mit einer Mischung aus Dampf und Luft. Der Katalysator ist Chrom/Aluminia-Typ. (US 5378350).

Die FBD Technik ist angelehnt an das Fluid Catalytic Cracking (FCC). Das System besteht aus einem Wirbelschichtreaktor und einem Regenerator. Die Wirbelschicht wird üblicherweise als blasenbildende Wirbelschicht betrieben, um die Verweilzeitverteilung der Edukte so klein wie möglich zu halten und trotzdem einen guten Wärmeübergang zu erzeugen. Einbauten im Reaktor können zusätzlich die Fluidisierung bzw. Rückvermischung bewusst stören. Der Cr/Al-Katalysator zirkuliert kontinuierlich im Gegenstrom zwischen Reaktor und Regenerator. Die Reaktionswärme (> 650 °C) wird durch die Wärmekapazität des heißen Katalysators erzeugt (US 5633421 und US 5656243).

Die bei der Dehydrierung erhaltenen Produktgemische werden üblicherweise mit aufwändigen Aufbereitungsverfahren in die gewünschten Produkte (Alkene), nicht umgesetzte Edukte sowie Nebenprodukte getrennt. Die Alkene können dann in diversen chemischen Prozessen als Ausgangsstoffe verwendet werden, so z. B. auch zur Herstellung von Aldehyden.

C₄ bzw. C₅-Aldehyde werden großtechnisch durch Hydroformylierung von Alkenen hergestellt. Das für diesen Zweck genutzte Propen bzw. Buten fällt in der Regel bei der Raffinierung bzw. beim Cracken von Erdöl an. Die Alkene sind dabei in der Regel keineswegs als Reinstoffe verfügbar sondern häufig in Begleitung weiterer Kohlenwasserstoffe anderer Substanzklassen und auch mit mehr oder weniger Kohlenstoffatomen. Zusätzlich enthalten die Alkene gegebenenfalls Butane, die als Inerte im Hydrofomylierungsprozess betrachtet werden und die Dimensionierung der Anlage vergrößern.

Die C₅-Aldehyde sind wichtige Ausgangsstoffe zur Erzeugung von Pentanolen, Pentansäuren und Pentylaminen. Durch Aldolkondensation und Totalhydrierung des Aldolkondensats können aus ihnen Decanole gewonnen werden, die Zwischenprodukte für die Herstellung von Weichmachern, Detergenzien und Schmiermitteln sind. Durch ihre Aldolkondensation, Hydrierung der olefinischen Doppelbindung des Aldolkondensats und anschließende Oxidation der aldehydischen Gruppe können Decansäuren erhalten werden, die beispielsweise zur Herstellung von Schmiermitteln oder Detergenzien verwendet werden können. In diesem Einsatzbereich ist es wichtig, dass die C₅-Aldehyde zu einem großen Anteil an der linearen Verbindung n-Pentanal bestehen bzw. der Anteil an verzweigten C₅-Aldehyden, wie insbesondere 2-Methylbutanal, möglichst gering ist.

Die Herstellung von n-Pentanal aus 2-Buten oder Gemischen davon durch isomerisierende Hydroformylierung wird in DE 101 08 474, DE 101 08 475, DE 101 08 476 und DE102 25 282 beschrieben. Die technischen Lehren aller dieser Schriften haben gemeinsam, dass in mindestens einem Hydroformylierungsschritt ein Rhodium aufweisendes, Katalysatorsystem mit einem Diphosphinliganden, der ein Xanthengerüst aufweist, verwendet wird. Mit diesem Katalysatorsystem können 2-Butene unter isomerisierenden Bedingungen hydroformyliert werden. Das Verhältnis von n-Pentanal zu 2-Methylbutanal liegt bei bestenfalls 85 zu 15. Die Schriften DE 101 08 474 und DE 101 08 475 beschreiben Verfahren, bei denen die Hydroformylierung zweistufig erfolgt. In der ersten Hydroformylierungsstufe wird unter Verwendung eines Katalysatorsystems, bestehend aus Rhodium und einem Monophosphin als Ligand, 1-Buten in einer Selektivität von 90 % zu n-Pentanal umgesetzt. Die nicht umgesetzten Butene, hauptsächlich 2-Butene, werden in der zweiten Hydroformylierungsstufe unter Verwendung des oben genannten Katalysatorsystems aus Rhodium/Bisphosphin umgesetzt. Die Schriften DE 101 08 476 und DE 102 25 282 beschreiben einstufige Hydroformylierungsverfahren.

Höhere Selektivitäten an n-Pentanal bei der Hydroformylierung von 2-Butenen können bei Verwendung eines Katalysatorsystems, bestehend aus Rhodium und sterisch anspruchsvollen aromatischen Bisphosphiten erhalten werden, wie sie beispielsweise in EP 0 213 639 beschrieben werden. Allerdings nimmt die Selektivität mit der Zeit stark ab.

Höhere Langzeitselektivität und geringere Zersetzungsrate des Katalysatorsystems wird erreicht, wenn das in EP 0 213 639 beschriebene Katalysatorsystem um ein sterisch gehindertes Amin ergänzt wird. Ein Verfahren unter Verwendung dieses Katalysatorsystems für die Hydroformylierung von 2-Buten zu Pentanalgemisch mit hohem n-Pentanalanteil wird in DE 10 2008 002187.3 offenbart. WO2004041703 beschreibt ein zweistufiges Verfahren zur Herstellung von Aldehyden aus Alkanen über Alkene mit verschiedenen Katalysatoren Problematisch an der Verwendung homogener Katalysatoren auf Basis von Rhodium bei der Hydroformylierung sind die Verluste an wertvollem Rhodium, die bei der Aufarbeitung der Produktströme erlitten werden. Vor diesem Hintergrund wurden Verfahren entwickelt, bei denen der Rhodium-Katalysator als heterogener bzw. quasi-heterogener Katalysator eingesetzt wurde. Die bislang aussichtsreichste Entwicklung ist die Hydroformylierung von Olefinen zu Aldehyden mittels sogenannter Supported-Ionic-Liquid-Phase-, kurz genannt SILP-Katalysatorsystemen. Dies sind katalytisch wirksame Zusammensetzungen in einem Mehrphasensystem, die ein festes Trägermaterial aufweisen, das mit einer ionischen Flüssigkeit umhüllt ist, der sogenannten Supported-Ionic-Liquid-Phase, kurz genannt SILP, in welcher der Übergangsmetall-, insbesondere Rhodium, aufweisende Katalysator enthalten ist. Einen Überblick über den Stand der Technik findet sich in WO 2012/041846, in der ebenfalls ein Verfahren, bei dem ein Rhodium-Komplexkatalysator in einer Phase aus ionischer Flüssigkeit aufgebracht auf einem festen Trägermaterial eingesetzt wird, beschrieben wird.

Literaturbekannte Ligandensysteme für die Gasphasenhydroformylierung als SILP-System sind u.a. Bisphosphite (DE 102010041821), Mono- und Bisphosphine (Riisager et al., Catal. Lett. 2003, 90, 149.; Riisager et al. J. Catal. 2003, 219, 452). Bisphosphite zeichnen sich durch eine hohe Langzeitstabilität von 700 h bei einer Reaktionstemperatur von 100 °C aus.

Obwohl im Stand der Technik bereits die Darstellung von Olefinen aus den entsprechenden Alkanen und die Herstellung von Aldehyden aus Olefinen beschrieben wurden, fehlt es an einfachen, wirtschaftlich anwendbaren Verfahren zur Herstellung von Aldehyden aus Alkanen und/oder Olefinen und Synthesegas.

Für den wirtschaftlichen Betrieb eines kontinuierlichen Verfahrens zur Hydroformylierung ist nicht allein die Nutzung eines sehr aktiven und selektiven Katalysatorsystems von Bedeutung. Besonders die Punkte Katalysatorrecycling -verbunden mit der Produktabtrennung - und Ligandenstabilität spielen eine entscheidende Rolle - nicht nur angesichts der hohen Rhodium- und Ligandenpreise, sondern auch des nur ansatzweise bekannten Einflusses von Verunreinigungen aus Liganden-Abbauprozessen auf die Aktivität und das Produktspektrum.

Ziel ist es die gesättigten Kohlenwasserstoffe in einem Verfahren in Aldehyde zu überführen bei einer gleichzeitigen besseren Abtrennbarkeit des Produktes vom Katalysator und einer besseren Rückgewinnung des Katalysators im Vergleich zum Stand der Technik.

Aufgabe der vorliegenden Erfindung war deshalb die Bereitstellung eines einfachen Verfahrens zur Herstellung von Aldehyden unter Verwendung von Alkanen als Ausgangsmaterial.

Überraschenderweise wurde gefunden, dass diese Aufgabe gelöst werden kann durch ein Verfahren, welches die Schritte: a) Dehydrieren mindestens eines Alkans in Gegenwart eines heterogenen Katalysators zu mindestens einem Alken, b) Umsetzen des Alkens oder der Alkene aus Schritt a) mit Wasserstoff und Kohlenmonoxid in Gegenwart eines heterogenen Katalysators, der von dem Katalysator in Schritt a) verschieden ist, aufweist, wobei als Katalysator in Schritt b) eine katalytisch wirksame Zusammensetzung eingesetzt wird, die einen festes Trägermaterial aufweist, welches eine ionische Flüssigkeit trägt, in welcher mindestens ein Übergangsmetall und mindestens ein Ligand aufweisender Katalysator enthalten ist.

Gegenstand der vorliegenden Erfindung ist deshalb ein Verfahren zur Herstellung von einem oder mehreren Aldehyden aus einem oder mehreren Alkanen, umfassend die Schritte: a) Dehydrieren mindestens eines Alkans in Gegenwart eines heterogenen Katalysators zu mindestens einem Alken, b) Umsetzen des Alkens oder der Alkene aus Schritt a) mit Wasserstoff und Kohlenmonoxid in Gegenwart eines heterogenen Katalysators, der von dem Katalysator in Schritt a) verschieden ist, welches dadurch gekennzeichnet ist, dass als Katalysator in Schritt b) eine katalytisch wirksame Zusammensetzung eingesetzt wird, die einen festes Trägermaterial aufweist, welches eine ionische Flüssigkeit trägt, in welcher ein mindestens ein Übergangsmetall und ein Ligand aufweisender Katalysator enthalten ist, wie in den Ansprüchen sowie der nachfolgenden Beschreibung beschrieben.

Ebenfalls Gegenstand der vorliegenden Erfindung ist eine Apparatur, geeignet zur Dehydrierung von mindestens einem Alkan zu mindestens einem Alken und Hydroformylierung des erhaltenen Alkens oder der erhaltenen Alkene zu Aldehyden, welche dadurch gekennzeichnet ist, dass sie mindestens zwei Reaktionszonen aufweist, die fluidleitend miteinander verbunden sind, wobei eine erste Reaktionszone als Festbett ausgeführt ist und einen Katalysator aufweist, der die Dehydrierung von Alkanen zu Alkenen katalysiert und eine zweite Reaktionszone als Festbett ausgeführt ist, die einen Katalysator aufweist, der die Hydroformylierung von Alkenen katalysiert und der ein festes Trägermaterial aufweist, welches eine ionische Flüssigkeit trägt, in welcher mindestens ein Übergangsmetall und mindestens ein Ligand enthalten ist, wie in den Ansprüchen sowie der nachfolgenden Beschreibung beschrieben.

Das erfindungsgemäße Verfahren und/oder die erfindungsgemäße Apparatur weisen einen oder mehrere der nachfolgenden Vorteile auf:
Mit dem in Schritt b) eingesetzten Katalysatorsystem auf Basis eines langzeitstabilen SILP-Systems ist die Heterogenisierung eines an und für sich homogenen Komplexkatalysators möglich. Somit ist eine einfachere Produktabtrennung und Rückgewinnung des Katalysators, insbesondere der darin enthaltenen Übergangsmetalle gegenüber homogenen Systemen möglich. Das erfindungsgemäße Verfahren ermöglicht den Einsatz von neuen Rohstoffquellen und die Nutzung von Alkanen, was zu einer deutlichen Wertschöpfung gegenüber der Nutzung von Olefinen führt. Mit dem erfindungsgemäßen Verfahren können, ausgehend von Alkanen, insbesondere Butan bzw. Alkan-, insbesondere Butan-haltigen Strömen in einfacher Weise die entsprechenden Aldehyde erzeugt werden. Auch bei Einsatz eines gemischten Feed-Stroms, der bereits Alkene enthält, erfolgt die Umsetzung selektiv. Außerdem kann mit dem erfindungsgemäßen Verfahren auf einen Aufreinigungsschritt nach der Dehydrierung verzichtet werden. Das erfindungsgemäße Verfahren kann zudem ohne den Einsatz von Lösemitteln auskommen. Die Aktivität ist vergleichbar mit der bei technischen Eduktströmen erreichbaren Aktivitäten.

Das erfindungsgemäße Verfahren und die erfindungsgemäß verwendete Apparatur werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt, insbesondere bezüglich der in Bezug genommenen Sachverhalte vollständig zum Offenbarungsgehalt der vorliegenden Erfindung gehören. Werden nachfolgend Angaben in Prozent gemacht, so handelt es sich, wenn nicht anders angegeben um Angaben in Gewichts-%. Werden nachfolgend Mittelwerte angegeben, so handelt es sich, wenn nicht anders angegeben um das Zahlenmittel. Werden nachfolgend Stoffeigenschaften, wie z. B. Viskositäten oder ähnliches angegeben, so handelt es sich, wenn nicht anders angegeben, um die Stoffeigenschaften bei 25 °C. Werden nachfolgend Messwerte angegeben, so wurden diese, wenn nicht anders angegeben, bei einem Umgebungsdruck von 1 bar und einer Umgebungstemperatur von 23 °C ermittelt. Werden in der vorliegenden Erfindung chemische (Summen-)Formeln verwendet, so können die angegebenen Indizes sowohl absolute Zahlen als auch Mittelwerte darstellen.

Das erfindungsgemäße Verfahren zur Herstellung von einem oder mehreren Aldehyden aus einem oder mehreren Alkanen, umfassend die Schritte:
a) Dehydrieren mindestens eines Alkans in Gegenwart eines heterogenen Katalysators zu mindestens einem Alken,
b) Umsetzen des Alkens oder der Alkene aus Schritt a) mit Wasserstoff und Kohlenmonoxid in Gegenwart eines heterogenen Katalysators, der von dem Katalysator in Schritt a) verschieden ist,
zeichnet sich dadurch aus, dass als Katalysator in Schritt b) eine katalytisch wirksame Zusammensetzung eingesetzt wird, die ein festes Trägermaterial aufweist, welches eine ionische Flüssigkeit trägt, in welcher ein mindestens ein Übergangsmetall und mindestens ein Ligand aufweisender Katalysator enthalten ist. Unter Tragen wird dabei insbesondere verstanden, dass das Trägermaterial auf der Oberfläche und/oder in den gegebenenfalls vorhandenen Poren eine ionische Flüssigkeit vorhanden ist, in der weitere Bestandteile vorhanden sein können. Die Menge der ionischen Flüssigkeit wird dabei vorzugsweise so gewählt, dass die ionische Flüssigkeit unter den Reaktionsbedingungen nicht vom Trägermaterial abgetragen (entfernt) wird.

Der Schritt b) des erfindungsgemäßen Verfahrens kann z. B. wie in WO 2012/041846 beschrieben durchgeführt werden. Das erfindungsgemäße Verfahren kann diskontinuierlich oder kontinuierlich durchgeführt werden. Vorzugsweise wird das erfindungsgemäße Verfahren kontinuierlich durchgeführt.

In Schritt a) können die Alkane als Reinstoffe oder Mischungen von Alkanen oder als Mischungen eingesetzt werden, die neben einem oder mehreren Alkanen weitere Bestandteile, wie z. B. ein oder mehrere Alkene, aufweisen. Die Alkane können aus fossilen Quellen, wie beispielsweise Erdöl oder Erdgas oder aus natürlichen Quellen, wie beispielsweise Biogas, oder Aufarbeitungsprozessen dieser Quellen stammen. Da Alkane häufig in Begleitung von Alkenen anfallen, kann es vorteilhaft sein, wenn in Schritt a) eine Mischung eingesetzt wird, die ein oder mehrere Alkane und ein oder mehrere Alkene und gegebenenfalls weitere Bestandteile aufweist. Wird in Schritt a) eine Mischung eingesetzt, die sowohl ein Alkan als auch ein Alken aufweist, so ist es vorteilhaft, wenn das Alkan bzw. die Alkane und das Alken bzw. die Alkene die identische Anzahl an Kohlenstoffatomen aufweist. Geeignete Mischungen sind z.B. sogenannte Raffinat-Ströme, die Iso-Butan, n-Butan, trans-2-Buten, 1-Buten, Iso-Buten, cis-2-Buten und Iso-Pentan in veränderlichen Zusammensetzungen aufweisen können.

Als Katalysatoren können in Schritt a) alle aus dem Stande der Technik bekannten Dehydrierungskatalysatoren, insbesondere die in den Eingangs bei der Beschreibung des Standes Technik der Dehydrierung genannten Literaturstellen genannten Dehydrierungskatalysatoren eingesetzt werden. Bevorzugte Katalysatoren sind solche, die Chrom, vorzugsweise als Chromoxid aufweisen.

Es kann vorteilhaft sein, wenn der in Schritt a) eingesetzt Katalysator ein Trägermaterial aufweist. Als Trägermaterialien werden vorzugsweise inerte und/oder poröse, bevorzugt inerte und poröse Trägermaterialien eingesetzt. Werden poröse Materialien eingesetzt so können diese mesoporöse Materialien, mikroporöse Materialien, makroporöse Materialien oder Schwammmaterialien sein. Bevorzugte Trägermaterialien sind ausgewählt aus der Gruppe umfassend Aluminiumoxid, Kieselsäure, Siliziumdioxid, Titandioxid, Zirkondioxid, Siliziumcarbid, Kohle, poröse Phosphate, poröse Polymere, Polymerschäume, Metallschäume, Metallorganische Gerüste, poröse Nitride, poröse Oxynitride, Aerogele auf Silikatbasis, und Mischungen aus diesen Komponenten. Als poröse Phosphate können Aluminiumphosphate, strukturmodifizierte Silikoaluminophosphate, wie z.B. SAPO-34, Verwendung finden. Als porösen Nitride oder porösen Oxynitride können z. B. Siliziumnitrid, Bornitrid, Kohlenstoffnitrid oder Metallorganische Gerüste Verwendung finden. Als mesoporöse Materialien können beispielsweise MCM-41-, MCM-48-, SBA-15-Schichtsilikate oder auch flammhydrolytisch hergestellte Silikate verwendet werden. Als mikroporöse Materialien können beispielsweise Zeolithe oder Alumosilikate verwendet werden. Besonders bevorzugt weist der in Schritt a) eingesetzte Katalysator Aluminiumoxid als Trägermaterial auf.

Das Trägermaterial, bevorzugt das Aluminiumoxidträgermaterial, weist vorzugsweise eine BET-Oberfläche von 50 bis 800 m² / g, bevorzugt von 100 bis 250 m² / g, besonders bevorzugt von 135 bis 170 m² / g und ganz besonders bevorzugt von 140 bis 160 m² / g auf. Das Trägermaterial, bevorzugt das Aluminiumoxidträgermaterial, weist vorzugsweise ein spezifisches Porenvolumen von 0,1 bis 2,5 cm³/g, bevorzugt von 0,25 bis 1 cm³/g, besonders bevorzugt von 0,4 bis 0,6 cm³/g auf. Vorzugsweise weist das Trägermaterial, bevorzugt das Aluminiumoxidträgermaterial, eine Kombination der vorgenannten bevorzugten Parameter aus BET-Oberfläche und spezifischem Porenvolumen auf, bevorzugt eine BET-Oberfläche von 125 - 170 m² / g und ein spezifisches Porenvolumen von 0,4 bis 0,6 cm³/g, wobei die Bestimmung dieser Werte nach der Hg-Methode gemäß DIN 66133 sowie der N₂-Adsorption nach DIN 66131 und DIN 66135 erfolgt.

Der in Schritt a) eingesetzt Katalysator, insbesondere der ein Trägermaterial aufweisende Katalysator kann in Form von Formkörpern, wie z. B. Extrudaten, eingesetzt werden.

Wird in Schritt a) ein Katalysator eingesetzt, der ein Trägermaterial aufweist, so beträgt der Chromgehalt bezogen auf die Summe aus Chrom(-verbindungen) und Trägermaterialien von 1 bis 25 Gew.-%, bevorzugt 5 bis 15 Gew.-%, besonders bevorzugt 9 bis 12 Gew.-%.

Der Schritt a) in dem erfindungsgemäßen Verfahren wird vorzugsweise bei Bedingungen durchgeführt, bei denen das Alkan oder die Alkane als Gase vorliegen. Werden in Schritt a) Mischungen eingesetzt, die Alkane aufweisen, so wird das erfindungsgemäße Verfahren vorzugsweise bei Bedingungen durchgeführt, bei denen mindestens 90 Gew.-% der eingesetzten Mischungen, bevorzugt 95 bis 100 Gew.-% und ganz besonders bevorzugt 100 Gew.-% der eingesetzten Mischung gasförmig vorliegen.

Der Schritt a) in dem erfindungsgemäßen Verfahren kann bei Unterdruck, Überdruck oder Normaldruck durchgeführt werden. Vorzugsweise wird der Schritt a) bei einem Druck von 0,1 bis 10 bar, bevorzugt von 0,5 bis 5 bar, besonders bevorzugt bei 0,1 bis 2 bar und ganz besonders bevorzugt bei 0,5 bis 1,5 bar.

Die Temperatur, bei der der Schritt a) durchgeführt wird beträgt vorzugsweise von 250 bis 650 °C, bevorzugt von 350 bis 550 °C, besonders bevorzugt 400 bis 500 °C und ganz besonders bevorzugt 425 bis 475 °C.

Vorzugsweise wird Schritt a) bei einer Kombination der genannten Drücke und Temperaturen, bevorzugt bei einer Kombination der bevorzugten und insbesondere besonders bevorzugten Drucke und Temperaturen durchgeführt. Ganz besonders bevorzugt wird Schritt a) deshalb bei einem Druck von 0,5 bis 1,5 bar und einer Temperatur von 425 bis 475 °C durchgeführt. Die vorgenannten bevorzugten Druck- und Temperaturbedingungen, bzw. die bevorzugten Kombinationen davon, werden vorzugsweise dann in Schritt a) verwendet, wenn als Alkan n-Butan oder als Gemisch ein n-Butan aufweisendes Gemisch eingesetzt wird.

Der Schritt b) des erfindungsgemäßen Verfahrens wird vorzugsweise in der Gasphase durchgeführt, das heißt, dass sich die Edukte während der Reaktion im gasförmigen Zustand befinden. Bevorzugte Temperaturen, bei denen Schritt b) durchgeführt wird, betragen vorzugsweise von 50 bis 200 °C, bevorzugt von 75 bis 140 °C und besonders bevorzugt von 80 bis 120 °C. Der Druck bei dem Schritt b) durchgeführt wird beträgt vorzugsweise von 2 bis 50 bar, bevorzugt von 5 bis 25 bar. Vorzugsweise wird Schritt b) bei einer Kombination der genannten Drucke und Temperaturen, bevorzugt bei einer Kombination der bevorzugten Drücke und Temperaturen durchgeführt. Ganz besonders bevorzugt wird Schritt b) deshalb bei 5 bis 10 bar und einer Temperatur von 80 bis 120 °C durchgeführt.

Das erfindungsgemäße Verfahren wird besonders bevorzugt so durchgeführt, dass Schritt a) bei einem Druck von 0,1 bis 10 bar und/oder, vorzugsweise und einer Temperatur von 250 bis 650 °C und Schritt b) in der Gasphase durchgeführt wird.

Als Übergangsmetall weist die in Schritt b) eingesetzte Zusammensetzung vorzugsweise solche der VIII. Gruppe des Periodensystems der Elemente, bevorzugt Kobalt, Rhodium, Iridium, oder Ruthenium, ganz besonders bevorzugt Rhodium auf. Die in Schritt b) eingesetzte katalytische Zusammensetzung weist das Übergangsmetall vorzugsweise in Form eines Komplexkatalysators auf. Bevorzugte Komplexkatalysatoren sind solche, die mindestens einen Liganden, ausgewählt aus der Gruppe umfassend Phosphine, Phosphite und Phosphonite, die bevorzugt jeweils mindestens einen dreiwertigen Phosphor aufweisen, aufweisen. Bevorzugt sind die Liganden ausgewählt aus Phosphiten und Mono- und Bisphosphinen. Besonders bevorzugt sind die Liganden ausgewählt aus der Gruppe der Phosphite und Bisphosphite.

Bevorzugt sind Bisphosphite ausgewählt aus: 2,2'-(3,3'-Di-tert-butyl-5,5'-dimethoxybiphenyl-2,2'-diyl)bis(oxy)bis(4,4,5,5-tetraphenyl-1,3,2-dioxaphospholan) und 6,6'-[(3,3'-Di-tert-butyl-5,5'-dimethoxy-1,1'-biphenyl-2,2'-diyl)bis(oxy)]bis(dibenzo[d,f][1,3,2]dioxaphosphepin), sowie Tridentate Phosphorliganden auf Basis von Antracentriol:. Die Herstellung der genannten Liganden kann z. B. wie in DE 102011085883A1 bzw. DE 102006058682 (US 2010036143) bzw. WO 2012/095253 und WO 2012/095255 und WO 2012/041846 beschrieben erfolgen.

Ionische Flüssigkeiten - nachfolgend IL genannt - sind im Rahmen der vorliegenden Erfindung niedrigschmelzende Salze, vorzugsweise Salze, die bei einem Druck von 1 bar eine Schmelztemperatur von kleiner 100 °C aufweisen, also bei einer Temperatur von kleiner 100 °C, vorzugsweise kleiner 50 °C und besonders bevorzugt kleiner-gleich 25 °C im flüssigen Aggregatzustand vorliegen und nahezu keinen messbaren Dampfdruck aufweisen, wie in Angew. Chem. Int. Ed. 2000, 39, 3772 - 3789 offenbart wird. Als ionische Flüssigkeiten können insbesondere jene verwendet werden, die in WO 2012/041846 beschrieben werden.

Bevorzugt werden als Ionische Flüssigkeit solche verwendet, bei denen das Anion ausgewählt ist aus der Gruppe umfassend: Tetrafluoroborat ([BF₄]⁻), Hexafluorophosphat ([PF₆]⁻), Dicyanamid ([N(CN)₂⁻]) , Bis(trifluoromethylsulfonyl)imid ([NTf₂]⁻), Tricyanomethid ([C(CN)₃]⁻) , Tetracyanoborat ([B(CN)₄]⁻), Halogenide (Cl⁻, Br⁻, F⁻, I⁻), Hexafluoroantimonat ([SbF₆]⁻), Hexafluoroarsenat ([AsF₆]⁻), Sulfat ([SO₄]²⁻) , Tosylat ([C₇H₇SO₃]⁻), Triflat (CF₃SO₃⁻), Nonaflat ([C₄F₉SO₃-), Tris-(Pentafluoroethyl)-trifluorophosphat ([PF₃(C₂F₅)₃]-), Thiocyanat ([SCN]⁻), Carbonat ([CO₃]²⁻), [R^{A}-COO]⁻, [R^{A}-SO₃]⁻ [R^{A}-SO₄]⁻ [R^{A}PO₄R^{B}]⁻ oder [(R^{A}-SO₂)₂N]⁻ mit R^{A} und R^{B} gleich oder ungleich, jeweils ein linearer oder verzweigter 1 bis 12 Kohlenstoffatome enthaltender aliphatischer oder alicyclischer Alkyl- oder Perfluoralkyl- oder ein C₅-C₁₈- substituierter Aryl-, C₅-C₁₈- substituierter Aryl-C₁-C₆-alkyl- oder C₁-C₆-Alkyl-C₅-C₁₈- substituierter Aryl-Rest ist, der durch Halogenatome substituiert ein kann;
und das Kation ausgewählt ist aus der Gruppe umfassend:
quaternäre Ammonium-Kationen der allgemeinen Formel [NRₐR_{b}R_{c}R_{d}]⁺ mit Rₐ, R_{b}, R_{c}, R_{d} ausgewählt aus C₁-C₈-Alkylgruppen;
Phosphonium-Kationen der allgemeinen Formel [PRₐR_{b}R_{c}R_{d}]⁺ mit Rₐ, R_{b}, R_{c}, R_{d} ausgewählt aus C₁-C₃-Alkylgruppen;
Imidazolium-Kationen der allgemeinen Formel (A) 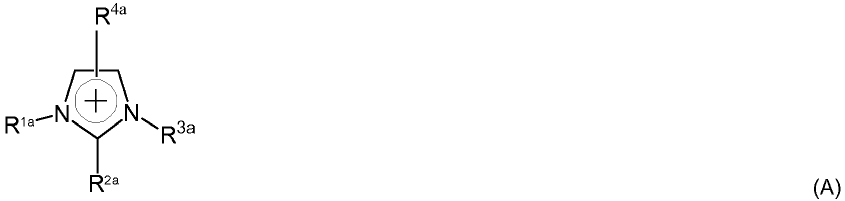
wobei der Imidazol-Kern substituiert sein kann mit wenigstens einer Gruppe R^{na} mit n =1, 2, 3 oder 4, die ausgewählt ist aus C₁-C₈-Alkyl-, C₁-C₆-Alkoxy-, C₁-C₆- substituierte Aminoalkyl-, C₅-C₁₂-substituierte Aryl- oder C₅-C₁₂- substituierte Aryl-C₁-C₆-Alkylgruppen;
Pyridinium-Kationen der allgemeinen Formel (B) 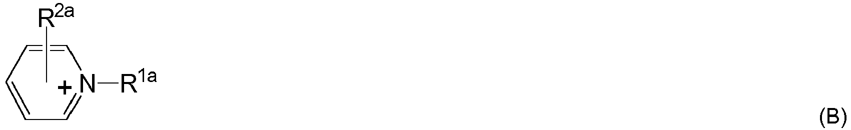
wobei der Pyridin-Kern substituiert sein kann mit wenigstens einer Gruppe R^{na} mit n = 1 oder 2, die ausgewählt ist aus C₁-C₆-Alkyl-, C₁-C₆-Alkoxy-, C₁-C₆- substituierte Aminoalkyl-, C₅-C₁₂-substituierte Aryl- oder C₅-C₁₂- substituierte Aryl-C₁-C₆-Alkylgruppen;
Pyrazolium-Kationen der allgemeinen Formel (C) 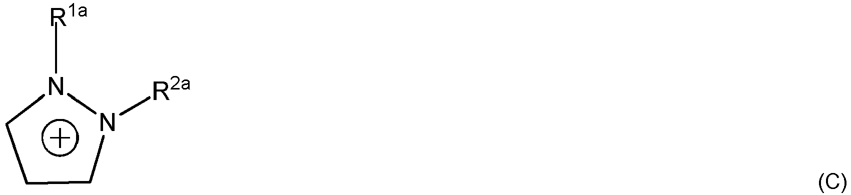
wobei der Pyrazol-Kern substituiert sein kann mit wenigstens einer Gruppe R^{na} mit n = 1 oder 2, die ausgewählt ist aus C₁-C₆-Alkyl-, C₁-C₆-Alkoxy-, C₁-C₆- substituierte Aminoalkyl-, C₅-C₁₂-substituierte Aryl- oder C₅-C₁₂- substituierte Aryl-C₁-C₆-Alkylgruppen;
Triazolium-Kationen der allgemeinen Formel (D) 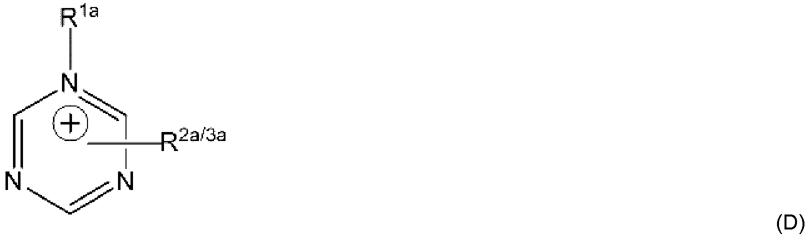
wobei der Triazol-Kern substituiert sein kann mit wenigstens einer Gruppe R^{na} mit n =1, 2, oder 3, die ausgewählt ist aus C₁-C₆-Alkyl-, C₁-C₆-Alkoxy-, C₁-C₆- substituierte Aminoalkyl-, C₅-C₁₂-substituierte Aryl- oder C₅-C₁₂- substituierte Aryl-C₁-C₆-Alkylgruppen.

Bevorzugt wird eine Ionische Flüssigkeit eingesetzt, die ausgewählt ist aus der Gruppe umfassend, vorzugsweise bestehend aus 1-Ethyl-3-methylimidazolium bis(trifluoromethylsulfonyl)imid, 1-Butyl-3-methylimidazolium hexafluorophosphat und 1-Butyl-3-methylimidazolium tetrafluoroborat. Besonders bevorzugt weist die in Schritt b) eingesetzte katalytische Zusammensetzung als ionische Flüssigkeit 1-Ethyl-3-methylimidazolium bis(trifluoromethylsulfonyl)imid ([EMIM] [NTf₂]) auf.

Als feste Trägermaterialien werden in dem erfindungsgemäßen Verfahren in den in Schritt b) eingesetzten Zusammensetzungen vorzugsweise inerte und/oder poröse, bevorzugt inerte und poröse Trägermaterialien eingesetzt. Werden poröse Materialien eingesetzt so können diese mesoporöse Materialien, mikroporöse Materialien, makroporöse Materialien oder Schwammmaterialien sein.

Bevorzugte in den in Schritt b) eingesetzten Zusammensetzungen vorhandene Trägermaterialien sind ausgewählt aus der Gruppe umfassend oder vorzugsweise bestehend aus Aluminiumoxid, Kieselsäure, Siliziumdioxid, Titandioxid, Zirkondioxid, Siliziumcarbid, Kohle, poröse Phosphate, poröse Polymere, Polymerschäume, Metallschäume, Metallorganische Gerüste, poröse Nitride, poröse Oxynitride, Aerogele auf Silikatbasis, und Mischungen aus diesen Komponenten. Besonders bevorzugt sind die Trägermaterialien ausgewählt aus solchen die Oxide des Siliziums, Aluminiums, Titans oder Zirkons oder Aktivkohle oder aus Mischungen aufweisen oder daraus bestehen. Als poröse Phosphate können Aluminiumphosphate, strukturmodifizierte Silikoaluminophosphate, wie z.B. SAPO-34, Verwendung finden. Als porösen Nitride oder porösen Oxynitride können z. B. Siliziumnitrid, Bornitrid, Kohlenstoffnitrid oder Metallorganische Gerüste Verwendung finden. Als mesoporöse Materialien können beispielsweise MCM-41-, MCM-48-, SBA-15-Schichtsilikate oder auch flammhydrolytisch hergestellte Silikate verwendet werden. Als mikroporöse Materialien können beispielsweise Zeolithe oder Alumosilikate verwendet werden. Ganz besonders bevorzugt weist die in Schritt b) eingesetzte katalytische Zusammensetzung als Trägermaterial ein Siliziumoxid auf. Die Herstellung geeigneter Trägermaterialien wird beispielsweise in WO 2012/041846 ausführlich beschrieben.

Bevorzugt in Schritt b) des erfindungsgemäßen Verfahrens eingesetzte Trägermaterialien weisen eine BET-Oberfläche von 100 bis 800 m²/g und/oder, bevorzugt und, ein Porenvolumen von 0,1 bis 2 ml/g auf. Besonders bevorzugt eingesetzte Trägermaterialien sind solche, Parameter in den genannten Bereichen liegen. Die Bestimmung dieser Werte erfolgt nach der Hg-Methode gemäß DIN 66133 sowie der N2-Adsorption nach DIN 66131 und DIN 66135.

Vorzugsweise werden als Katalysator bzw. katalytisch wirksame Zusammensetzungen in Schritt b) solche eingesetzt wie sie z. B. in WO 2012/041846, DE 102010041821 und Riisager et al., Catal. Lett. 2003, 90, 149.; Riisager et al. J. Catal. 2003, 219, 452 und in den nachfolgend beschriebenen Beispielen beschrieben werden.

Es kann vorteilhaft sein, wenn die Zusammensetzung in Schritt b) ein organisches Amin aufweist, welches von den IL verschieden ist. Durch die Verwendung solcher organischer Amine als Stabilisatoren kann die Langzeitstabilität der Zusammensetzung gesteigert werden. Bevorzugt umfasst das verwendete organische Amin mindestens einen Rest mit einer 2,2,6,6-Tetramethylpiperidineinheit nach Formel (E):

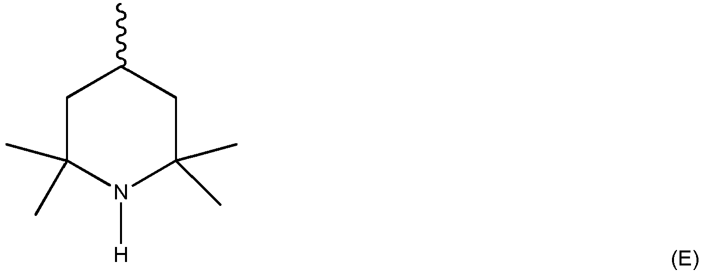

Besonders bevorzugt ist das organische Amin in der erfindungsgemäßen Zusammensetzung ausgewählt aus den Verbindungen der Formeln (Ea) - (Eh): 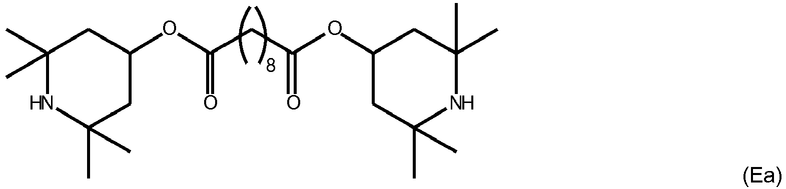 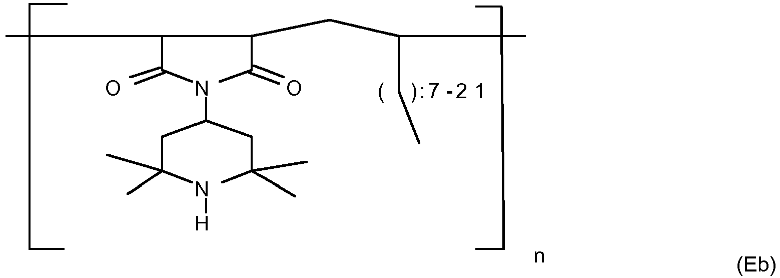 mit n = 1 bis 20 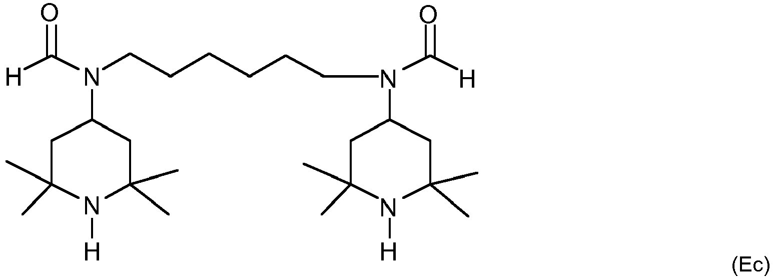 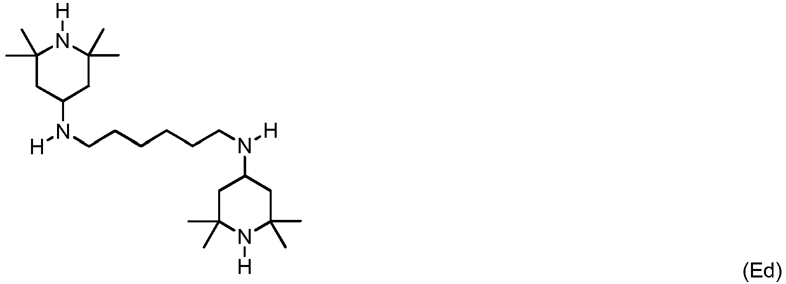 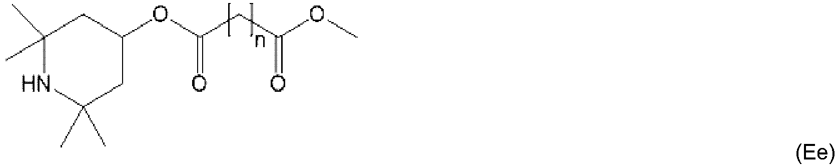 mit n = 1 bis 12 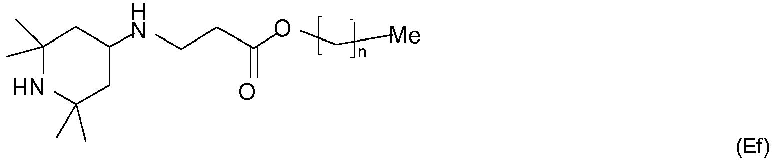 mit n = 1 bis 17 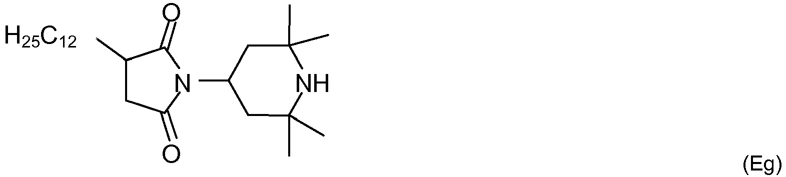 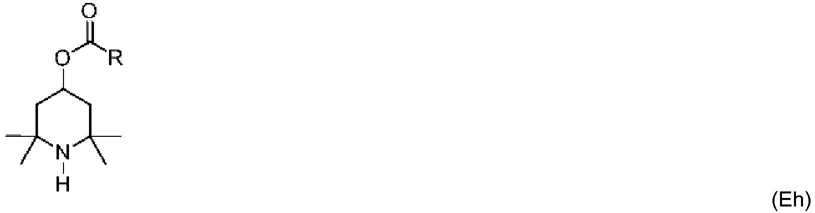 mit R = C₆- bis C₂₀-Alkyl.

Die in Schritt b) eingesetzte katalytische Zusammensetzung kann auf jede bekannte Weise durch Mischen der Komponenten hergestellt werden. Vorzugsweise wird die erfindungsgemäße Zusammensetzung durch das nachfolgend beschriebene Verfahren hergestellt bzw. ist dadurch erhältlich. Dieses zeichnet sich dadurch aus, dass es die Schritte:
i) vorlegen einer Vorstufe mindestens einer Verbindung eines Übergangsmetalls, vorzugsweise eines Metalls aus der VIII. Nebengruppe des Periodensystems der Elemente;
ii) in-Kontakt-bringen mindestens einer Verbindung eines Übergangsmetalls, vorzugsweise eines Metalls aus der VIII. Nebengruppe des Periodensystems der Elemente mit einem molaren Überschuss an mindestens einer Phosphorhaltigen organischen Verbindung, unter Verwendung eines inerten Lösungsmittels;
iii) Zugabe mindestens eines festen Trägermaterials zu der unter ii) generierten Mischung;
iv) Zugabe mindestens einer Ionischen Flüssigkeit und optional weiterer Verbindungen, wie z. B. Stabilisatoren (org. Amine) und
v) entfernen des inerten Lösungsmittels unter Erhalt der Zusammensetzung, wobei bevorzugt die Schritte i) bis iv) in beliebiger Reihenfolge durchgeführt und wobei bevorzugt in Schritt i) mindestens eine Verbindung eines Metalls aus der VIII. Nebengruppe in einem inerten Lösungsmittel vorgelegt werden kann.

Das erfindungsgemäße Verfahren kann insbesondere dann vorteilhaft eingesetzt werden, wenn es sich bei dem Alkan um Cₙ-Alkane (n=3, 4 oder 5) oder deren Mischungen handelt, bei dem Alken um Cₙ-Alkene (n= 3, 4, 5) und deren Mischungen und bei dem Aldehyd um Cₙ₊₁-Aldehyde und deren Mischungen handelt.

Das erfindungsgemäße Verfahren kann in jeder geeigneten Apparatur durchgeführt werden. Vorzugsweise wird das erfindungsgemäße Verfahren in einer erfindungsgemäßen Apparatur durchgeführt.

Die erfindungsgemäße Apparatur, geeignet zur Dehydrierung von einem oder mehreren Alkanen zu einem oder mehreren Alkenen und Hydroformylierung des erhaltenen Alkens oder der erhaltenen Alkene zu Aldehyden, zeichnet sich dadurch aus, dass sie mindestens zwei Reaktionszonen aufweist, die fluidleitend miteinander verbunden sind, wobei eine erste Reaktionszone als Festbett ausgeführt ist und einen Katalysator aufweist, der die Dehydrierung von Alkanen zu Alkenen katalysiert, und eine zweite Reaktionszone als Festbett ausgeführt ist, die einen Katalysator aufweist, der die Hydroformylierung von Alkenen katalysiert und der ein festes Trägermaterial aufweist, welches eine ionische Flüssigkeit trägt, in welcher mindestens ein Übergangsmetall und mindestens ein Ligand enthalten ist, wie er in den vorangegangenen Abschnitten ausführlich beschrieben wurde. Unter fluidleitend verbunden wird verstanden, dass ein Fluid, welches aus einer ersten Reaktionszone als Produkt erhalten wird, zumindest teilweise in die zweite Reaktionszone gelangen kann. Die Festbetten mit den Katalysatoren können auf bekannte Arten und Weisen in den Reaktoren angeordnet sein.

Es kann vorteilhaft sein, wenn zwischen den Reaktionszonen mindestens ein Mittel zur Kondensation zumindest eines Teils des aus der Reaktionszone erhaltenen Produktstroms vorhanden ist. Es kann ebenfalls vorteilhaft sein, wenn zwischen den Reaktionszonen mindestens ein Mittel zur Verdampfung mindestens eines Teils des in die zweite Reaktionszone geführten Eduktstroms vorhanden ist. Vorzugsweise ist in der erfindungsgemäßen Apparatur zwischen den Reaktionszonen mindestens ein Mittel zur Kondensation zumindest eines Teils des aus der Reaktionszone erhaltenen Produktstroms vorhanden ist und daran anschließend mindestens ein Mittel zur Verdampfung mindestens eines Teils des kondensierten Produktstroms angeordnet ist.

In der erfindungsgemäßen Apparatur kann zwischen den Reaktionszonen zumindest ein Mittel vorhanden sein, welches es ermöglicht einen Teil des aus der ersten Reaktionszone erhaltenen Produktstroms abzutrennen. Auf diese Weise können z. B. nicht umgesetzte Alkane ganz oder teilweise aus dem Produktstrom abgetrennt werden und z. B. in die erste Reaktionszone zurückgeführt oder ausgeschleust werden.

Die Reaktionszonen sind bevorzugt in unterschiedlichen Reaktoren angeordnet. Diese Ausführungsart hat den Vorteil, dass die ggf. zwischen den Reaktionszonen verbauten Mittel, wie z. B. Mittel zur Verdampfung, zur Kondensation, zum Fördern, wie z. B. Pumpen, zum Abtrennen, wie z. B. Scheidebehälter, o.ä. in Form von Standardapparaturen zum Aufbau der erfindungsgemäßen Apparatur verwendet werden können. Zudem können auf einfache Weise Möglichkeiten geschaffen werden, bei Ausfall eines Reaktors / einer Reaktorzone einen Weiterbetrieb der Anlage sicherzustellen, in dem ein oder mehrere Reservereaktoren vorgehalten werden, die im Bedarfsfall zugeschaltet werden.

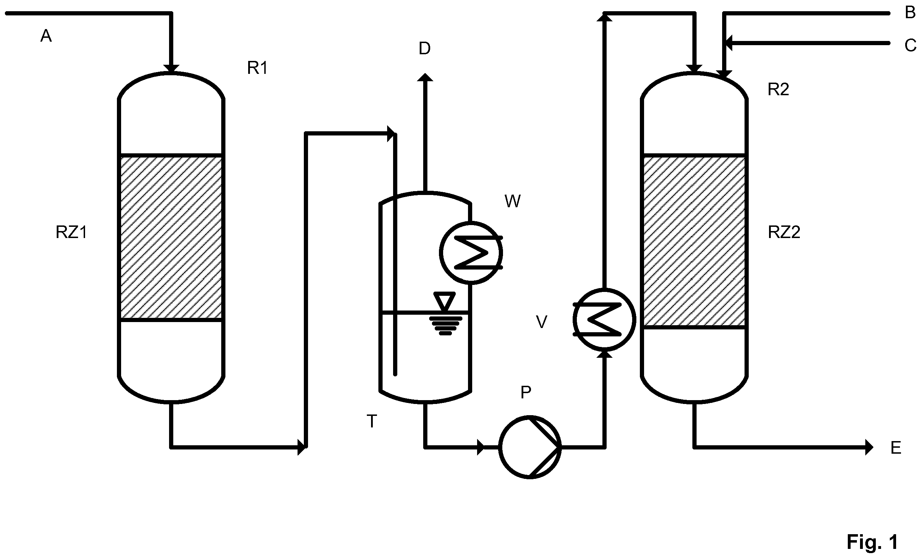

Die vorliegende Erfindung wird an Hand der Figur 1 näher erläutert ohne darauf beschränkt zu sein. Fig. 1 zeigt schematisch den Aufbau einer erfindungsgemäß verwendeten Apparatur. Darin bezeichnet A den Alkan-haltigen Zustrom zu Reaktor R1, der eine Reaktionszone RZ1 aufweist, die als Festbett einen Dehydrierkatalysator aufweist. Der Produktstrom aus Reaktor R1 wird einem Tank T zugeführt, der mit einem Wärmetauscher W ausgerüstet ist. Über die Leitung D können gasförmige Stoffe, z. B. Wasserstoff sowie ggf. Alkane, aus der Apparatur entfernt werden. Über die Pumpe P wird der Inhalt des Tanks T über den Verdampfer V zumindest teilweise dem Reaktor R2 zugeführt, der eine Reaktionszone RZ2 aufweist, die in einem Festbett eine katalytisch wirksame Zusammensetzung aufweist, die ein festes Trägermaterial aufweist, welches eine ionische Flüssigkeit trägt, in welcher ein mindestens ein Übergangsmetall und mindestens ein Ligand aufweisender Katalysator enthalten ist. Über Rohrleitung B kann Wasserstoff und über Rohrleitung C kann Kohlenmonoxid dem Reaktor R2 zugeführt werden.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

### Generell verwendete Arbeitstechniken:

Alle Luft- und Feuchtigkeitsempfindlichen Substanzen wurde in einer inerten Atmosphäre unter Argon (4.6, Linde AG, entspr. 99,996 Vol.-%) gelagert. Die Katalysatorpräparationen wurden in einer Glove-Box durchgeführt. Schlenk-Technik wurde für Arbeiten außerhalb der Glove-Box verwendet. Hygroskopische Substanzen wurden vor dem Einsatz unter Hochvakuum für mindestens 24 Stunden getrocknet. Die Reinheit der Industriegase wird mit Hilfe von Codewerten ausgedrückt. Dabei bezeichnet die erste Ziffer die Anzahl der "Neuner", die zweite Ziffer ist die erste von "Neun" abweichende Dezimalstelle.

### Einsatzstoffe:

### Gase

Für die Dehydrierung wurde n-Butan (Linde AG, 2.5, entspr. 99,5 Vol.-%) verwendet. Synthetische Luft mit einem Anteil von 20 Vol.-% Sauerstoff wurden zur Regenerierung des heterogenen Katalysators eingesetzt. Die Hydroformylierung wurde ausschließlich in der Gasphase durchgeführt. Synthesegas wurde durch Kohlenstoffmonoxid (Linde AG, 3.7, entspr. 99,97 Vol.%) und Wasserstoff (Linde AG, 5.0, entspr. 99,999 Vol.-%) bereitgestellt. Das molare Verhältnis von CO zu H2 betrug 1:1. Für alle Anlagenversuche wurde Helium (Linde AG, 4.6, entspr. 99,996 Vol.-%) zur Inertisierung verwendet.

### Heterogener Katalysator für die Dehydrierung von Alkanen

Für die Alkan-Dehydrierung wurde ein heterogener Katalysator (11 % Cr/Al2O3) von Evonik Industries AG eingesetzt. Der Katalysator hat einen Chromgehalt von 11 Gew.-% (6,7 Gew.-% CrO₃ (Chrom mit einer Oxidationsstufe von +6 als Chrom(VI) bezeichnet) und 11 Gew.-% Cr₂O₃ Chrom mit einer Oxidationsstufe von +3 als Chrom(III) bezeichnet) auf Al₂O₃ (3 mm Extrudate, BET-Oberfläche 147 m²/g, spez. Porenvolumen 0,48 cm³/g, wobei die Bestimmung dieser Werte nach der Hg-Methode gemäß DIN 66133 sowie der N₂-Adsorption nach DIN 66131 und DIN 66135 erfolgt. Die Analytik des gesamten Chromgehalts erfolgt über quantitative Röntgenfloureszensanalyse. Die quantitative Chrom(VI)-Analytik wurde über ein fotometrisches Verfahren (Chrom (VI) mittels Diphenylcarbazit) nach alkalischer Extraktion durchgeführt. Chrom(IV) wird danach zunächst aus dem mittels einer NaOH-/Na2CO3-Lösung ausgelaugt. Das Eluat wird vereinigt und der Chrom (VI)-Gehalt nach DIN 38406 D 24 bestimmt. Die Bestimmung des Chrom (III)-Gehalts erfolgt aus der Differenz des gesamten Chromgehaltes und des Chrom (VI)-Gehaltes.

### Herstellung des heterogenen Katalysators für die Dehydrierung von Alkanen:

1600 g Al₂O₃ Träger werden mit einer Lösung aus 400 g CrO₃ in 864 ml VE-Wasser in einer 10 L Drageetrommel bei Raumtemperatur mit einer Drehgeschwindigkeit von 31 min-1 für 5 Minuten besprüht. Die Trocknung des Katalysators erfolgt im 5 L Wirbelbettreaktor unter 7,5 m³ Luft/h mit 1 K/Minute von Raumtemperatur auf 110 °C und einer Haltedauer der genannten Bedinungen von 7 h. Anschließend erfolgt ein Kalzinierungsschritt indem von 110 °C mit 2 K/Minute auf 450 °C an Luft erhitzt wird und 5 Stunden bei 450 °C gehalten wird.

### Zusammensetzung der SILP-Katalysatoren

Die SILP-Katalysatoren wurden unter Verwendung der in Tabelle 1 angegebenen Substanzen hergestellt.

**Tabelle 1: Übersicht über die verwendeten Substanzen**

| Funktion | Name | Abkürzung | Molekulargewicht (g mol⁻¹) | CAS-Nummer |
|---|---|---|---|---|
| Präkursor | (Acetylacetonat)dicarbonylrhodium(I) | Rh(CO)₂(aca c) | 258,03 | 14874-82-9 |
| Ligand | 2,2'-(3,3'-Di-tert-butyl-5,5'-dimethoxybiphenyl-2,2'-diyl)bis(oxy)bis(4,4,5,5-tetraphenyl-1,3,2-dioxaphospholan) | Benzpinakol (BzP) | 1147,29 | |
| Ligand | 6,6'-[(3,3''-Di-*tert*-butyl-5,5'-dimethoxy-1,1'-biphenyl-2,2'-diyl)bis(oxy)]bis(dibenzo[*d*,*f*][1,3,2]dio xaphosphepin) | Biphephos (BP) | | 121627-17-6 |
| IL | 1-Ethyl-3-methylimidazolium bis(trifluoromethylsulfonyl)imid | [EMIM][NTf2] | 391,31 | 174899-82-2 |
| Stabilisator | Bis(2,2,6,6-tetramethyl-4-piperidyl)sebacat | Sebacat | 480,72 | 52829-07-9 |
| Träger | Kieselgel 100 0,063 - 0,200 mm | SiO₂ | 60,08 | 7631-86-9 |

### SILP-Katalysator 1

Präkursor für den SILP-Katalysator war (Acetylacetonat)dicarbonylrhodium(I) Rh(CO)₂acac. Der Diphosphitligand 2,2'-(3,3'-Di-tert-butyl-5,5'-dimethoxybiphenyl-2,2'-diyl)bis(oxy)bis(4,4,5,5-tetraphenyl-1,3,2-dioxaphospholan) der Formel (I) 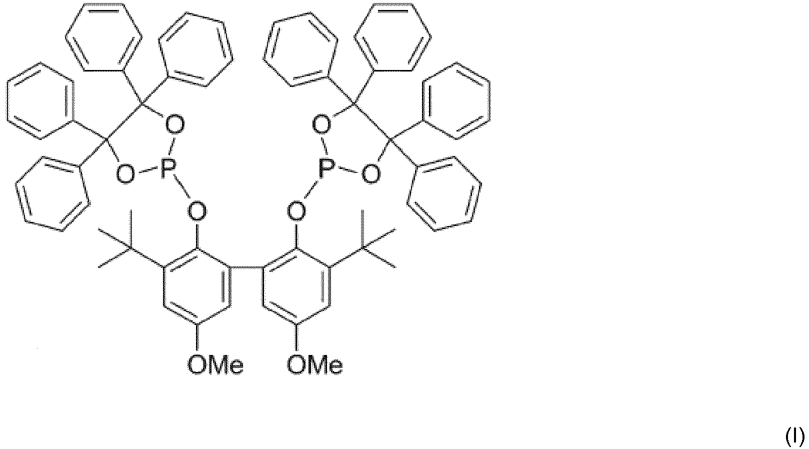 wurde, wie in DE 102006058682 (US 2010036143) beschrieben, synthetisiert. Die ionische Flüssigkeit (IL) 1-Ethyl-3-methylimidazolium bis(trifluoromethylsulfonyl)imid ([EMIM] [NTf₂]) wurde von Merck KGaA bezogen. Als Trägermaterial für die SILP-Katalysatoren wurde Kieselgel 100 der Merck KGaA eingesetzt. Die Zugabe der Ionischen Flüssigkeit, im folgenden IL, erfolgte in der Art, dass ein Beladungsgrad α derart eingestellt wird, dass er einen Wert von 0,1 bzw. 10 Vol.-% annimmt. Unter einem Beladungsgrad α versteht man im Zusammenhang mit SILP-Katalysatorsystemen das Verhältnis von dem Volumen unter Normalbedingungen der jeweils verwendeten Ionischen Flüssigkeit IL zu dem Porenvolumen des jeweils verwendeten Trägermaterials. Das Massenverhältnis von Rh zu Trägermaterial wurde so eingestellt, dass es 0,2 Gew.-% Rh bezogen auf das Trägermaterial betrug. Bei der Synthese des Komplexkatalysators wurde soviel Präkursor und Ligand eingesetzt, dass das molare Verhältnis von Ligand zu Rhodium 10 zu 1 betrug. Die Menge an Stabilisator wurde so gewählt, dass das molare Verhältnis von Stabilisator zu Ligand 4 zu 1 betrug.

### SILP-Katalysator 2

Präkursor für den SILP-Katalysator war (Acetylacetonat)dicarbonylrhodium(I) Rh(CO)₂acac. Der Ligand BP (Biphephos) 6,6'-[(3,3'-Di-*tert*-butyl-5,5-dimethoxy-1,1'-biphenyl-2,2'-diyl)bis(oxy)]bis(dibenzo[*d,f*][1,3,2]dioxaphosphepin), CAS 121627-17-6 der Formel (II) 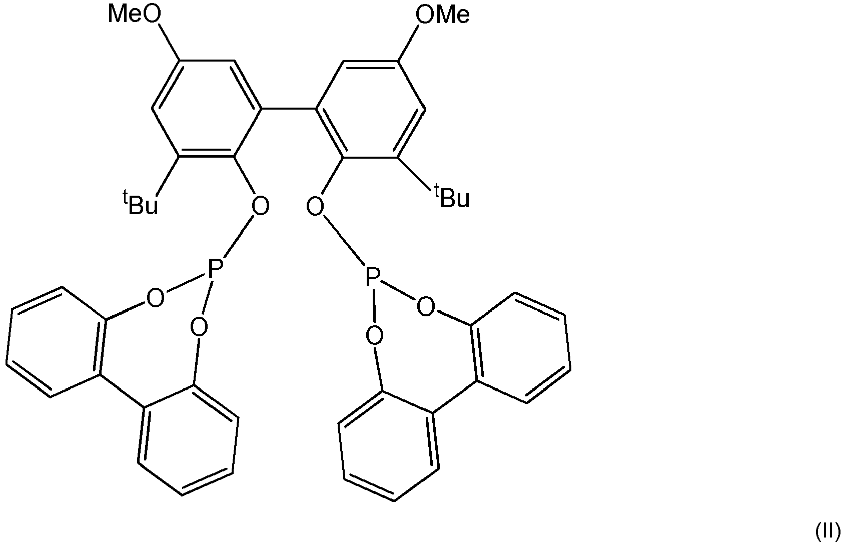 wurde analog WO 2012/095253 bzw. WO 2012/095255 synthetisiert. Die IL 1-Ethyl-3-methylimidazolium bis(trifluoromethylsulfonyl)imid ([EMIM] [NTf₂]) wurde von Merck KGaA bezogen. Als Trägermaterial für die SILP-Katalysatoren wurde Kieselgel 100 der Merck KGaA eingesetzt.

### Herstellung der SILP-Katalysatoren

Die SiO₂-Träger wurden vor der Verwendung unter Stickstoff (Linde AG, 5.0) mit folgendem Temperaturprogramm kalziniert: Der Träger wurde von Raumtemperatur auf 150 °C mit einer Heizrampe von 2 °C min⁻¹ aufgeheizt und anschließend zwei Stunden konstant bei 150 °C gehalten. In einer zweiten Heizphase wurde der Träger mit einer Heizrampe von 2,5 °C min⁻¹ auf 600 °C erhitzt. Für weitere 12 Stunden wurde der Träger konstant bei 600 °C gehalten, um eine fast vollständige Decarboxylierung der Oberfläche zu gewährleisten. Die aktive Abkühlphase auf 50 °C unter Stickstoff wurde mit einer Rampe von bei 5 °C min⁻¹ durchgeführt, um eine Recarboxylierung des hygrokopischen Materials zu verhindern. Anschließend wurde der Träger für 24 Stunden evakuiert und in der Glove-Box bis zur Verwendung gelagert. Die morphologischen Eigenschaften der Träger wurden mittels Stickstoff-Gasadsorption mit dem Gerät "Quantrachrome Quadrasorb Sl" bestimmt und sind in Tabelle 2 angegeben.

**Tabelle 2: Morphologische Eigenschaften des SiO₂ -Träger**

| Träger | Material | Partikelgröße (mm) | Oberfläche^{a} nach BET (M² g⁻¹) | Porenvolumen^{b} (cm³ g⁻¹) | Mittlere Porenweite (nm) |
|---|---|---|---|---|---|
| Kieselgel 100 | SiO2 | 0,063 - 0,2 | 361 | 1,02 | 10,5 |

| | | | | | |
|---|---|---|---|---|---|
| ^{a}bestimmt durch BET Adsorptionsisotherme, ^{b}BJH kumulatives Porenvolumen | | | | | |

Die Präparation der SILP-Katalysatoren erfolgte durch Versetzen der porösen Trägermaterialien mit einer Lösung aus Katalysatorkomplex, ionischer Flüssigkeit und Stabilisator. Zur Lösung der Komponenten wurde Dichlormethan (Merck KGaA, getrocknet, max. 0,004 % H₂O) als Lösungsmittel eingesetzt. Nachdem das Lösemittel durch Einengen am Rotationsverdampfer wieder entfernt worden war, wurde der SILP-Katalysator als rieselfähiges Pulver erhalten. Um eine Zersetzung des Katalysators durch Luftsauerstoff auszuschließen, wurden die Einwaagen der einzelnen Komponenten ausschließlich in der Glove-Box durchgeführt.

SILP-Katalysator 1 wurde, wie in M. Jakuttis, A. Schönweiz, S. Werner, R. Franke, K.-D. Wiese, M. Haumann, P. Wasserscheid, 'Rhodium-Phosphite SILP Catalysis for the Highly Selective Hydroformylation of Mixed C4 Feedstocks', Angew. Chem., Int. Ed. 2011, 50, 4492-4495 beschrieben, mit einem Stabilisator präpariert. Bei dem Stabilisator handelt sich um das sterisch gehinderte Diamin Bis(2,2,6,6-tetramethyl-4-piperidyl)sebacat.

### Konzeption der Versuchsanlage

Zur Durchführung einer kontinuierlichen Kaskadenreaktion wurde eine Versuchsanlage konzeptioniert und aufgebaut. Die Gesamtanlage setzt sich aus der Steuerung, dem Reaktionsteil und der online Analytik zusammen. Der Reaktionsteil gliederte sich zwei wesentliche Untersektionen. Eine Sektion für die katalytische Alkandehydrierung und eine Sektion für die SILPkatalysierte Hydroformylierung. Figur 1 gibt den schematischen Aufbau der Versuchsanlage wieder.

Die Dosierung der gasförmigen Komponenten für die Alkandehydrierung erfolgte über 4 Massendurchflussregler der Baureihe "EL-Flow" (Mass Flow Controller - MFC) der Firma Bronkhorst High-Tech B.V. Neben dem Reaktionsgas n-Butan konnte Helium zum Spülen des Reaktors und zur Partialdruckvariation eingesetzt werden. Synthetische Luft und Wasserstoff wurden zur Regenerierung des heterogenen Katalysators angebracht. Um eine gleichzeitige Förderung von Wasserstoff und synthetischer Luft in den Reaktor zu vermeiden, war ein Dreiwegehahn installiert, der nur für eines der beiden Gase offen steht. Zusätzlich wurden zwei pneumatische Ventile über ein elektropneumatisches Wegeventil manuell angesteuert, das ebenfalls ein gleichzeitiges Ausströmen unterbindet.

Der Dehydrierungsreaktor bestand aus einer hochwarmfesten Nickel-Chrom-Eisen-Legierung (2.4816, Alloy 600). Der Festbettreaktor hatte eine Länge von 650 mm, einen Innendurchmesser von 10 mm und eine Wandstärke von 7,5 mm. Der Reaktor ist bis zu einer Temperatur von 850 °C bei Atmosphärendruck vom TÜV-Süd zugelassen. Die Katalysator-schüttung lag auf einem Bett aus Glaswolle auf: Die Glaswolle lag wiederum auf einer gesinterten Metallfritte aus 1.4571 auf, die passgenau 50 mm vor dem Reaktorausgang eingesetzt war. Die Fritte besaß eine mittlere Porenweite von 6 µm. Zusätzlich war in der Schüttung ein Thermoelement Pt-100 eingebracht. 2/3 der Länge des Reaktors waren als Vorheizstrecke ausgelegt, um das Reaktionsgas auf Temperaturen bis zu 850 °C vor der Schüttung aufzuheizen. Der Rohrreaktor verfügte über einen Heizmantel mit drei Heizzonen, die unabhängig voneinander geregelt werden konnten. Aufgrund der Elastizitätsgrenze Rp0,2 und der Temperaturstabilität von 1.4571 werden die unteren 50 mm der Rohrreaktors nicht aktiv beheizt.

Die kohlenwasserstoffhaltigen Edukte wurden dem Hydroformylierungsteil aufgrund des Dampfdruckes bei Raumtemperatur flüssig über eine HPLC-Pumpe (Dr. Ing. Herbert Knauer GmbH "Smartline 100") zudosiert. Die HPLC-Pumpe war mit federgestützten Kugelventilen ausgerüstet, um eine besser Förderkonstanz zu gewährleisten. Synthesegas wurde über jeweils einen MFC für Kohlenstoffmonoxid und Wasserstoff bereitgestellt. Helium konnte über einen MFC oder über ein Nadelventil in die Anlage dosiert werden. Helium konnte zum Spülen der Anlage, zum Prüfen auf Leckagen oder zum Verdünnen der Reaktionsmischung verwendet werden. Der Vorheizer erhitzte die Flüssigkeit knapp unterhalb der Siedetemperatur bei Betriebsdruck. Im Mischer werden die Kohlenwasserstoffe durch Reduzierung des Partialdruckes mit Synthesegas in die Gasphase überführt.

Der Hydroformylierungsreaktor war aus dem Werkstoff 1.4571 gefertigt. Der Reaktor war bei 50 bar bis 450 °C zugelassen. Die Länge des Reaktors betrug 500 mm, der Innendurchmesser 10 mm und die Wandstärke 5 mm. Die SILP-Katalysatorschüttung lag auf einer Fritte mit 6 µm mittlerer Porenweite auf. Ein Thermoelement war in die Schüttung eingebracht. Ein zweites Thermoelement war 50 mm über der Fritte in die Wandung des Reaktors eingefasst. Die Regelung der Einzonenheizung erfolgte über das Thermoelement in der Wandung des Reaktors.

Die Kondensationsbehälter hatten jeweils ein Volumen von 0,6 Litern. Die Steuerung der pneumatischen Ventile war darauf ausgelegt, dass die Behälter im Wechsel befüllt und entleert werden konnten. Schwinggabelsonden am Kopf und am Boden der Behälter gewährleisteten kondensierte Flüssigkeit und verhinderten ein Volllaufen bzw. ein vollständiges Entleeren der Behälter. Digitale Drucksensoren waren in die Notabschaltung integriert. Die pneumatischen Ventile, die digitalen Druckaufnehmer und die Füllstandsensoren wurden über eine Steuerung des Typs "LOGO!" der Siemens AG gesteuert. Die Kühlung der Behälter erfolgte mit einem luftgekühlten Kryostat "MPC-K6s" der Firma Peter Huber Kältemaschinebau GmbH, der bis -25 °C mit einer Glysantin/Wasser-Mischung betrieben werden konnte. Der Kryostat hatte eine Kälteleistung von 0,05 kW bei -20 °C. Für eine Verbesserung des Wärmeübergangs und eine Vergrößerung der Austauschfläche waren Hohlzylinder aus Edelstahl als regellose Schüttung in die Behälter eingebracht. Die Hohlzylinder hatten eine Länge von 15 mm und einen Durchmesser von 10 mm. Um ein Fehlsignal der Schwinggabelsonden durch die Füllkörper zu vermeiden, waren diese mit einem stabilen Drahtgitter umgeben.

Rohrleitungen und Behälter waren aus Edelstahl 1.4571 und die Ventilkörper aus Edelstahl 1.4401 gefertigt. Die Ventile enthielten Packungen entweder aus dem Werkstoff Polytetrafluorethylen (PTFE, Tmax = 232 °C) oder aus dem thermoplastischen Kunststoff Polyetherketon (PEEK, Tmax = 315 °C). Die Rohrleitungen wurden über anliegende Heizbänder der Firma Horst GmbH temperiert. Die Isolation der Leitungen wurde mit einem Band aus Glaswolle bewerkstelligt.

Die Analyse der Gasströme beider Reaktorstränge erfolgte alternierend oder seriell. Hierzu wurde ein 4-Port SD (Selector, Dead-End) Ventil von Valco Instruments Co. Inc. über eine Relaissteuerung an die Schnittstelle des Gaschromatographen angeschlossen. Somit konnte die Ansteuerung des Ventils in die Steuerungssoftware des GCs implementiert werden. Die Gaszusammensetzung wurde mit einem Gaschromatographen "GC 450" von Bruker Corporation durchgeführt.

Die gesamte Anlage war mit Sicherheitseinrichtungen ausgestattet, die einen sicheren und kontinuierlichen Betrieb über mehrere Tage ohne Anwesenheit eines Betreibers gewährleisten können. Überdruck, Übertemperatur, Ausfall einer Heizschnur oder eines Reglers versetzten die Anlage in einen sicheren Zustand.

### Gaschromatographie

Die Analyse der Reaktionsgase erfolgte mit einem Gaschromatographen "GC 450" der Firma Bruker Corporation. Der Gaschromatograph (GC) war mit einem Wärmeleitfähigkeitsdetektor (WLD) und zwei Flammenionisationsdetektoren (FID) ausgestattet. Die Aufgabe der Proben auf die Trennsäulen erfolgte gleichzeitig über zwei pneumatische Einspritzventile mit 250 µl Probenschleifen. Argon wurde als Trägergas verwendet. Wasserstoff, Kohlenstoffmonoxid und Helium wurde durch eine WLD-Analyse quantifiziert. Über den EFC-24-Front (Electronic Flow Control) und PR-1 (Pressure Regulator) wurden der Säulendruck und die Trägergasgeschwindigkeit auf den Säulen geregelt. Der eingestellt Säulendruck betrug für den EFC-24 3,45 bar für eine Minute und 2,41 bar für die restliche Messung. Der PR-1 war konstant 3,17 bar eingestellt. Eine Hayesep Q80/100 (0,5 m x 1/16" x 1,0 mm) Säule trennt die Permanentgase von den kohlenwasserstoffhaltigen Stoffen. Eine Trennsäule vom Typ Shincarbon Micropacked trennt letztlich Wasserstoff, Kohlenmonoxid und Helium auf.

Die kohlenwasserstoffhaltigen Stoffe wurden mit zwei Flammenionisationsdetektoren analysiert. Das Splitverhältnis von Trägergas zu Probenvolumen lag konstant bei 20 : 1. Für EFC-21 wurde ein konstanter Fluss von 2,5 ml min-1 und für EFC-24-Rear ein konstanter Überdruck von 0,31 bar eingestellt. Mit einer CP-Wax (25 m x 0,53 mm x 2 µm) Trennsäule wurden zuerst Alkane und Alkene von Aldehyden, Alkoholen und Aldolprodukten getrennt. Alkane und Alkene wurden auf die HP-Plot-S (50 m x 0,53 mm x 15 µm) Trennsäule geleitet und Aldehyde, Alkohole und Aldolprodukte wurden auf der Deactivated Fused Silica (10 m x 0,32 mm) weiter aufgetrennt. Plot-Aluminiumoxid-Säulen besitzen eine hohe Auflösung für Kohlenwasserstoffisomere, können aber durch Moleküle mit funktionellen Gruppe deaktiviert werden.

Mit der folgenden GC-Methode konnte eine vollständige Trennung der Komponenten erreicht werden. Die Anfangstemperatur von 55 °C wurde für 4 Minuten gehalten. Anschließend folgte eine lineare Aufheizphase mit 4,5 °C min-1 auf 100 °C. 100 °C wurden für 12 Minuten konstant gehalten. Die letzte Aufheizphase mit 10 °C min-1 auf 130 °C dient zur Entfernung hochsiedender Verbindungen bzw. zur Verkürzung derer Retentionszeit. Die Säulen wurden für 6 Minuten konstant bei 130 °C belassen. Die Gesamtdauer der Methode betrug 35 Minuten. Die Temperatur der Einhausung des Wärmeleitfähigkeitsdetektors betrug 220 °C und die des Filamentes 350 °C. Die zwei Flammenionisationsdetektoren waren auf 250 °C beheizt.

### Beispiel 1:

Der Versuch wurde in einer wie oben beschriebenen Versuchsanlage, die in der Dehydrierungssektion mit 10 g des Dehydrierungskatalysators und in der Hydroformylierungssektion mit 3 g SILP-Katalysator 1 beschickt war, durchgeführt. Die Reaktion in der Dehydrierungssektion wurde bei einem Druck von 1 bar bei einer Temperatur von 450 °C durchgeführt. Die Hydroformylierung wurde bei einem Druck von 10 bar und einer Temperatur von 100 °C durchgeführt. Der gasförmige Edukt-Zustrom (Butan) zur Hydroformylierung wurde auf 43,4 Nml min⁻¹ eingestellt. Der Zustrom an Synthesegas wurde im Bereich von 21,7 bis 86,8 Nml min⁻¹ eingestellt. Die Resultate des Versuchs sind in Tabelle 3 angegeben.

### Beispiel 2:

Der Versuch gemäß Beispiel 1 wurde wiederholt, mit dem Unterschied, dass SILP-Katalysator 2 an Stelle des SILP-Katalysators 1 verwendet wurde. Die Ergebnisse sind in Tabelle 3 angegeben.

### Beispiel 3:

Der Versuch gemäß Beispiel 1 wurde wiederholt, wobei als Eduktstrom ein Raffinat III Strom eingesetzt wurde, der die nachfolgende Zusammensetzung in mol-% aufwies: Iso-Butan: 0,18, n-Butan: 25,09, trans-2-Buten: 25,3, 1-Buten: 31,5, Iso-Buten: 0,11, cis-2-Buten: 14,22 und Iso-Pentan: 3,6. Die Ergebnisse sind in Tabelle 3 angegeben.

**Tabelle 3: Ergebnisse der Beispiele 1 bis 3**

| Beispiel | Raumzeitausbeute in g n-Pentanal/(kg SILP-Katalysator)*h | n-/iso-Selektivität in % |
|---|---|---|
| Bsp. 1 | 35 - 60 | > 99 |
| Bsp. 2 | 20 - 120 | 96 - 98 |
| Bsp. 3 | 290 - 320 | > 99 |

Wie den Versuchen der Beispiele 1 bis 3 entnommen werden kann, können mit dem erfindungsgemäßen Verfahren ausgehend von Alkanen, insbesondere Butan bzw. Alkan-, insbesondere Butan-haltigen Strömen in einfacher Weise die entsprechenden Aldehyde erzeugt werden. Auch bei Einsatz eines gemischten Feed-Stroms, der bereits Alkene enthält, erfolgt die Umsetzung selektiv. Außerdem kann mit dem erfindungsgemäßen Verfahren auf einen Aufreinigungsschritt nach der Dehydrierung sowie auf den Einsatz von Lösemitteln verzichtet werden. Die n-/iso-Selektivität ist vergleichbar mit der bei technischen Eduktströmen erreichbaren Aktivitäten.

## Patentansprüche

1. Verfahren zur Herstellung von einem oder mehreren Aldehyden aus einem oder mehreren Alkanen, umfassend die Schritte:
a) Dehydrieren mindestens eines Alkans in Gegenwart eines heterogenen Katalysators zu mindestens einem Alken,
b) Umsetzen des Alkens oder der Alkene aus Schritt a) mit Wasserstoff und Kohlenmonoxid in Gegenwart eines heterogenen Katalysators, der von dem Katalysator in Schritt a) verschieden ist,
**dadurch gekennzeichnet, dass** als Katalysator in Schritt b) eine katalytisch wirksame Zusammensetzung eingesetzt wird, die ein festes Trägermaterial aufweist, welches eine ionische Flüssigkeit trägt, in welcher ein mindestens ein Übergangsmetall und mindestens ein Ligand aufweisender Katalysator enthalten ist.

2. Verfahren nach Anspruch 1, **dadurch gekennzeichnet, dass** in Schritt a) eine Mischung eingesetzt wird, die Alkane und Alkene aufweist.

3. Verfahren nach Anspruch 1 oder 2, **dadurch gekennzeichnet, dass** in Schritt a) ein Katalysator eingesetzt wird, der Chrom aufweist.

4. Verfahren nach einem der Ansprüche 1 bis 3, **dadurch gekennzeichnet, dass** Schritt a) bei 0,1 bis 10 bar und/oder einer Temperatur von 250 bis 650 °C und Schritt b) in der Gasphase durchgeführt wird.

5. Verfahren nach mindestens einem der Ansprüche 1 bis 4, **dadurch gekennzeichnet, dass** die in Schritt b) das Übergangsmetall ausgewählt ist aus Kobalt, Rhodium, Iridium und Ruthenium.

6. Verfahren nach mindestens einem der Ansprüche 1 bis 5, **dadurch gekennzeichnet, dass** der Ligand ausgewählt ist aus der Gruppe umfassend die Phosphine, Phosphite, Phosphonite, Biphosphite und Bisphosphine.

7. Verfahren nach Anspruch 6, **dadurch gekennzeichnet, dass** es sich bei dem Liganden um einen tridentaten Phosphorliganden auf Basis von Antracentriol oder um 2,2'-(3,3'-Di-tert-butyl-5,5'-dimethoxybiphenyl-2,2'-diyl)bis(oxy)bis(4,4,5,5-tetraphenyl-1,3,2-dioxaphospholan) oder um 6,6'-[(3,3'-Di-tert-butyl-5,5'-dimethoxy-1,1'-biphenyl-2,2'-diyl)bis(oxy)]bis(dibenzo[d,f][1,3,2]dioxaphosphepin) handelt.

8. Verfahren nach einem der Ansprüche 1 bis 7, **dadurch gekennzeichnet, dass** die in Schritt b) eingesetzte katalytische Zusammensetzung als ionische Flüssigkeit mindestens eine ionische Flüssigkeit, ausgewählt aus der Gruppe der ionischen Flüssigkeiten, bei denen das Anion ausgewählt ist aus der Gruppe umfassend: Tetrafluoroborat ([BF₄]⁻), Hexafluorophosphat ([PF₆]⁻), Dicyanamid ([N(CN)₂]⁻), Bis(trifluoromethylsulfonyl)imid ([NTf₂]⁻), Tricyanomethid ([C(CN)₃]⁻), Tetracyanoborat ([B(CN)₄]⁻), Halogenide (Cl⁻, Br⁻, F⁻, I⁻), Hexafluoroantimonat ([SbF₆]⁻), Hexafluoroarsenat ([AsF₆]⁻), Sulfat ([SO₄]²⁻) , Tosylat ([C₇H₇SO₃]⁻), Triflat (CF₃SO₃⁻), Nonaflat ([C₄F₉SO₃-), Tris-(Pentafluoroethyl)-trifluorophosphat ([PF₃(C₂F₅)₃]-), Thiocyanat ([SCN]⁻), Carbonat ([CO₃]²⁻), [R^{A}-COO]⁻, [R^{A}-SO₃]⁻, [R^{A}-SO4]⁻, [R^{A}PO₄R^{B}]⁻ oder [(R^{A}-SO₂)₂N]⁻ mit R^{A} und R^{B} gleich oder ungleich, jeweils ein linearer oder verzweigter 1 bis 12 Kohlenstoffatome enthaltender aliphatischer oder alicyclischer Alkyl- oder Perfluoralkyl- oder ein C₅-C₁₈- substituierter Aryl-, C₅-C₁₈- substituierter Aryl-C₁-C₆-alkyl- oder C₁-C₆-Alkyl-C₅-C₁₈- substituierter Aryl-Rest ist, der durch Halogenatome substituiert ein kann,und das Kation ausgewählt ist aus der Gruppe umfassend:
quaternäre Ammonium-Kationen der allgemeinen Formel [NRₐR_{b}R_{c}R_{d}]⁺ mit Rₐ, R_{b}, R_{c}, R_{d} ausgewählt aus C₁-C₈-Alkylgruppen;
Phosphonium-Kationen der allgemeinen Formel [PRₐR_{b}R_{c}R_{d}]⁺ mit Rₐ, R_{b}, R_{c}, R_{d} ausgewählt aus C₁-C₈-Alkylgruppen;
Imidazolium-Kationen der allgemeinen Formel (A) 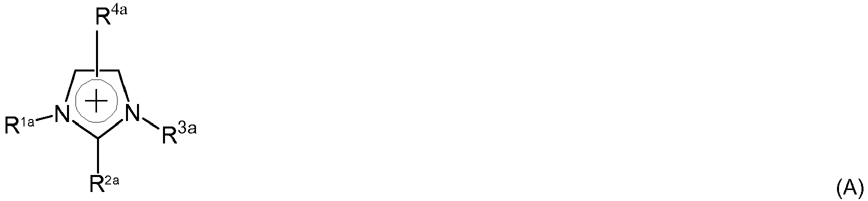
wobei der Imidazol-Kern substituiert sein kann mit wenigstens einer Gruppe R^{na} mit n =1, 2, 3 oder 4, die ausgewählt ist aus C₁-C₈-Alkyl-, C₁-C₆-Alkoxy-, C₁-C₆- substituierte Aminoalkyl-, C₅-C₁₂- substituierte Aryl- oder C₅-C₁₂- substituierte Aryl-C₁-C₆-Alkylgruppen;
Pyridinium-Kationen der allgemeinen Formel (B) 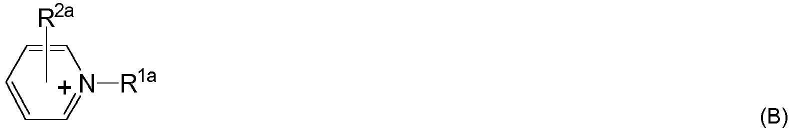
wobei der Pyridin-Kern substituiert sein kann mit wenigstens einer Gruppe R^{na} mit n = 1 oder 2, die ausgewählt ist aus C₁-C₆-Alkyl-, C₁-C₆-Alkoxy-, C₁-C₆- substituierte Aminoalkyl-, C₅-C₁₂- substituierte Aryl- oder C₅-C₁₂- substituierte Aryl-C₁-C₆-Alkylgruppen;
Pyrazolium-Kationen der allgemeinen Formel (C) 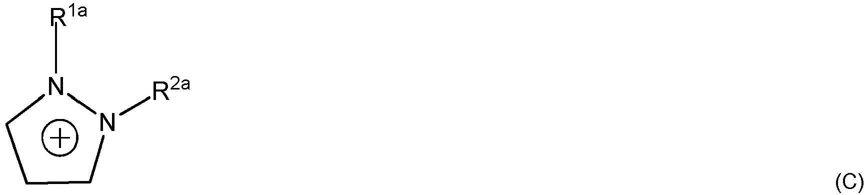
wobei der Pyrazol-Kern substituiert sein kann mit wenigstens einer Gruppe R^{na} mit n = 1 oder 2, die ausgewählt ist aus C₁-C₆-Alkyl-, C₁-C₆-Alkoxy-, C₁-C₆- substituierte Aminoalkyl-, C₅-C₁₂- substituierte Aryl- oder C₅-C₁₂- substituierte Aryl-C₁-C₆-Alkylgruppen;
Triazolium-Kationen der allgemeinen Formel (D) 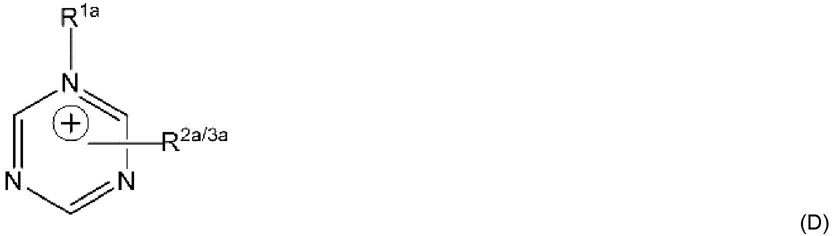
wobei der Triazol-Kern substituiert sein kann mit wenigstens einer Gruppe R^{na} mit n =1, 2, oder 3, die ausgewählt ist aus C₁-C₆-Alkyl-, C₁-C₆-Alkoxy-, C₁-C₆- substituierte Aminoalkyl-, C₅-C₁₂- substituierte Aryl- oder C₅-C₁₂- substituierte Aryl-C₁-C₆-Alkylgruppen.

9. Verfahren nach Anspruch 8, **dadurch gekennzeichnet, dass** eine ionische Flüssigkeit ausgewählt aus der Gruppe umfassend 1-Ethyl-3-methylimidazolium bis(trifluoromethylsulfonyl)imid ([EMIM] [NTf₂]), 1-Butyl-3-methylimidazolium hexafluorophosphat und 1-Butyl-3-methylimidazolium tetrafluoroborat,eingesetzt wird..

10. Verfahren nach einem der vorhergehenden Ansprüche, **dadurch gekennzeichnet, dass** die Zusammensetzung in Schritt b) ein organisches Amin aufweist, welches von der eingesetzten ionischen Flüssigkeit verschieden ist.

11. Verfahren nach einem der vorhergehenden Ansprüche, **dadurch gekennzeichnet, dass** die in Schritt b) eingesetzte katalytische Zusammensetzung als Trägermaterial ein Oxid von Aluminium, Silizium, Titan oder Zirkon oder Aktivkohle oder Mischungen davon aufweist.

12. Verfahren nach einem der vorhergehenden Ansprüche, **dadurch gekennzeichnet, dass** es sich bei dem Alkan um ein oder mehrere Cₙ-Alkane (n=3, 4 oder 5) oder deren Mischungen handelt, bei dem Alken um Cₙ-Alkene (n= 3, 4, 5) und deren Mischungen handelt.

## Claims

1. Process for preparing one or more aldehydes from one or more alkanes, comprising the steps of:
a) dehydrogenating at least one alkane in the presence of a heterogeneous catalyst to afford at least one alkene,
b) reacting the alkene or alkenes from step a) with hydrogen and carbon monoxide in the presence of a heterogeneous catalyst distinct from the catalyst in step a),
**characterized in that** the catalyst employed in step b) is a catalytically active composition comprising a solid support material carrying an ionic liquid comprising a catalyst comprising at least one transition metal and at least one ligand.

2. Process according to Claim 1, **characterized in that** step a) comprises employing a mixture comprising alkanes and alkenes.

3. Process according to either of Claims 1 and 2, **characterized in that** step a) comprises employing a catalyst comprising chromium.

4. Process according to any of Claims 1 to 3, **characterized in that** step a) is carried out at from 0.1 to 10 bar and/or at a temperature of from 250°C to 650°C and step b) is carried out in the gas phase.

5. Process according to at least one of Claims 1 to 4, **characterized in that** in step b) the transition metal is selected from cobalt, rhodium, iridium and ruthenium.

6. Process according to at least one of Claims 1 to 5, **characterized in that** the ligand is selected from the group comprising phosphines, phosphites, phosphonites, biphosphites and biphosphines.

7. Process according to Claim 6, **characterized in that** the ligand is a tridentate phosphorus ligand based on anthracenetriol or is 2,2'-(3,3'-di-tert-butyl-5,5'-dimethoxybiphenyl-2,2'-diyl)bis(oxy)bis(4,4,5,5-tetraphenyl-1,3,2-dioxaphospholane) or is 6,6'-[(3,3'-di-tert-butyl-5,5'-dimethoxy-1,1'-biphenyl-2,2'-diyl)bis(oxy)]bis(dibenzo[d,f][1,3,2]dioxaphosphepi ne) .

8. Process according to any of Claims 1 to 7, **characterized in that** the catalytic composition employed in step b) comprises as ionic liquid at least one ionic liquid selected from the group of ionic liquids whose anion is selected from the group comprising: tetrafluoroborate ([BF₄]⁻), hexafluorophosphate ([PF₆]⁻), dicyanamide ([N(CN)₂]⁻), bis(trifluoromethylsulphonyl)imide ([NTf₂]⁻), tricyanomethide ([C(CN)₃]⁻), tetracyanoborate ([B(CN)₄]⁻), halides (Cl⁻, Br⁻, F⁻, I⁻), hexafluoroantimonate ([SbF₆]⁻), hexafluoroarsenate ([AsF₆]⁻), sulphate ([SO₄]²⁻), tosylate ([C₇H₇SO₃]⁻), triflate (CF₃SO₃⁻), nonaflate ([C₄F₉SO₃-), tris(pentafluoroethyl)trifluorophosphate ([PF₃(C₂F₅)₃]-), thiocyanate ([SCN]⁻), carbonate ([CO₃]²⁻), [R^{A}-COO]⁻, [R^{A}-SO₃]⁻, [R^{A-}SO₄]⁻, IR^{A}PO₄R^{B}]⁻or [(R^{A}-SO₂)₂N]⁻ where R^{A} and R^{B} are identical or different and are each a linear or branched, 1 to 12 carbon-atom-containing, aliphatic or alicyclic alkyl or perfluoroalkyl radical or a C₅-C₁₈-substituted aryl, C₅-C₁₈-substituted aryl-C₁-C₆-alkyl or C₁-C₆-alkyl-C₅-C₁₈-substituted aryl radical, which may be substituted by halogen atoms, and whose cation is selected from the group comprising:
quaternary ammonium cations of general formula [NRₐR_{b}R_{c}R_{d}]⁺ where Rₐ, R_{b}, R_{c}, R_{d} are selected from C₁-C₈-alkyl groups;
phosphonium cations of general formula [PRₐR_{b}R_{c}R_{d}]⁺ where Rₐ, R_{b}, R_{c}, R_{d} are selected from C₁-C₈-alkyl groups;
imidazolium cations of general formula (A) 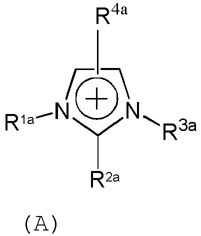
where the imidazole ring may be substituted by at least one R^{na} group, where n =1, 2, 3 or 4, selected from C₁-C₈-alkyl, C₁-C₆-alkoxy, C₁-C₆-substituted aminoalkyl, C₅-C₁₂-substituted aryl or C₅-C₁₂-substituted aryl-C₁-C₆-alkyl groups;
pyridinium cations of general formula (B) 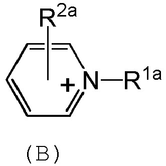
where the pyridine ring may be substituted by at least one R^{na} group, where n =1 or 2, selected from C₁-C₆-alkyl, C₁-C₆-alkoxy, C₁-C₆-substituted aminoalkyl, C₅-C₁₂-substituted aryl or C₅-C₁₂-substituted aryl-C₁-C₆-alkyl groups;
pyrazolium cations of general formula (C) 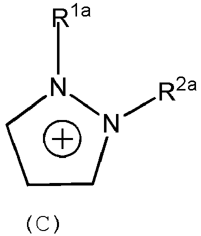
where the pyrazole ring may be substituted by at least one R^{na} group, where n =1 or 2, selected from C₁-C₆-alkyl, C₁-C₆-alkoxy, C₁-C₆-substituted aminoalkyl, C₅-C₁₂-substituted aryl or C₅-C₁₂-substituted aryl-C₁-C₆-alkyl groups;
triazolium cations of general formula (D) 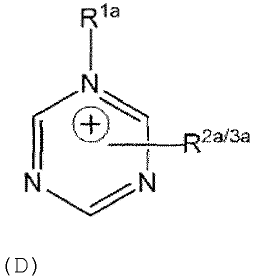
where the triazole ring may be substituted by at least one R^{na} group, where n = 1, 2 or 3, selected from C₁-C₆-alkyl, C₁-C₆-alkoxy, C₁-C₆-substituted aminoalkyl, C₅-C₁₂-substituted aryl or C₅-C₁₂-substituted aryl-C₁-C₆-alkyl groups.

9. Process according to Claim 8, **characterized in that** an ionic liquid selected from the group comprising 1-ethyl-3-methylimidazolium bis(trifluoromethylsulfonyl)imide ([EMIM] [NTf₂]), 1-butyl-3-methylimidazolium hexafluorophosphate and 1-butyl-3-methylimidazolium tetrafluoroborate is employed.

10. Process according to any preceding claim, **characterized in that** the composition in step b) comprises an organic amine distinct from the ionic liquid employed.

11. Process according to any preceding claim, **characterized in that** the catalytic composition employed in step b) comprises as support material an oxide of aluminium, silicon, titanium or zirconium or activated carbon or mixtures thereof.

12. Process according to any preceding claim, **characterized in that** the alkane represents one or more Cₙ alkanes (n=3, 4 or 5) or mixtures thereof, the alkene represents Cₙ alkenes (n=3, 4 or 5) and mixtures thereof.

## Revendications

1. Procédé pour la préparation d'un ou de plusieurs aldéhydes à partir d'un ou de plusieurs alcanes, comprenant les étapes :
a) déshydrogénation d'au moins un alcane en présence d'un catalyseur hétérogène en au moins un alcène,
b) transformation de l'alcène ou des alcènes de l'étape a) avec de l'hydrogène et du monoxyde de carbone en présence d'un catalyseur hétérogène, qui est différent du catalyseur dans l'étape a),
**caractérisé en ce qu'**on utilise, comme catalyseur dans l'étape b), une composition catalytiquement active qui présente un matériau support solide, qui porte un liquide ionique, dans lequel est contenu un catalyseur présentant au moins un métal de transition et au moins un ligand.

2. Procédé selon la revendication 1, **caractérisé en ce qu'**on utilise, dans l'étape a), un mélange qui présente des alcanes et des alcènes.

3. Procédé selon la revendication 1 ou 2, **caractérisé en ce qu'**on utilise, dans l'étape a), un catalyseur qui présente du chrome.

4. Procédé selon l'une quelconque des revendications 1 à 3, **caractérisé en ce que** l'étape a) est réalisée à 0,1 jusqu'à 10 bars et/ou à une température de 250 à 650°C et l'étape b) est réalisée en phase gazeuse.

5. Procédé selon au moins l'une quelconque des revendications 1 à 4, **caractérisé en ce que** le métal de transition dans l'étape b) est choisi parmi le cobalt, le rhodium, l'iridium et le ruthénium.

6. Procédé selon au moins l'une quelconque des revendications 1 à 5, **caractérisé en ce que** le ligand est choisi dans le groupe comprenant les phosphines, les phosphites, les phosphonites, les biphosphites et les bis-phosphines.

7. Procédé selon la revendication 6, **caractérisé en ce qu'**il s'agit, pour les ligands, d'un ligand de phosphore tridentate à base d'anthracène-triol ou de 2,2'-(3,3'-di-tert-butyl-5,5'-diméthoxybiphényl-2,2'-diyl)bis(oxy)bis(4,4,5,5-tétraphényl-1,3,2-dioxaphospholane) ou de 6,6'-[(3,3'-di-tert-butyl-5,5'-diméthoxy-1,1'-biphényl-2,2'-diyl)bis(oxy)]bis(dibenzo[d,f][1,3,2]dioxaphosphépine).

8. Procédé selon l'une quelconque des revendications 1 à 7, **caractérisé en ce que** la composition catalytique utilisée dans l'étape b) contient, comme liquide ionique, au moins un liquide ionique, choisi dans le groupe des liquides ioniques dans lesquels l'anion est choisi dans le groupe comprenant : le tétrafluoroborate ([BF₄]⁻), l'hexafluorophosphate ([PF₆]⁻), le dicyanamide ([N(CN)₂]⁻), le bis(trifluorométhylsulfonyl)imide ([NTf₂]⁻), le tricyanométhide ([C(CN)₃]⁻), le tétracyanoborate ([B(CN)₄]⁻), les halogénures (Cl⁻, Br⁻, F⁻, I⁻), l'hexafluoroantimonate ([SbF₆]⁻), l'hexafluoroarsénate ([AsF₆]⁻), le sulfate ([SO₄]²⁻), le tosylate ([C₇H₇SO₃]⁻), le triflate (CF₃SO₃⁻), le nonaflate ([C₄F₉SO₃⁻), le tris-(pentafluoroéthyl)-trifluorophosphate ([PF₃(C₂F₅)₃]⁻), le thiocyanate ([SCN]⁻), le carbonate ([CO₃]²⁻), [R^{A}-COO]⁻, [R^{A}-SO₃]⁻, [R^{A}-SO₄]⁻, [R^{A}PO₄R^{B}]⁻ ou [(R^{A}-SO₂)₂N]⁻, où R^{A} et R^{B}, identiques ou différents, représentent à chaque fois un radical alkyle ou perfluoroalkyle aliphatique ou alicyclique linéaire ou ramifié, comprenant 1 à 12 atomes de carbone ou un radical C₅-C₁₈-aryle substitué, un radical (C₅-C₁₈-aryle substitué)-C₁-C₆-alkyle ou un radical (C₁-C₆-alkyle substitué)-C₅-C₁₈-aryle, qui peut être substitué par des atomes d'halogène et le cation est choisi dans le groupe comprenant :
les cations d'ammonium quaternaire de formule générale [NRₐR_{b}R_{c}R_{d}]⁺ dans laquelle Rₐ, R_{b}, R_{c}, R_{d} sont choisis parmi les groupes C₁-C₈-alkyle ;
les cations de phosphonium de formule générale [PRₐR_{b}R_{c}R_{d}]⁺ dans laquelle Rₐ, R_{b}, R_{c}, R_{d} sont choisis parmi les groupes C₁-C₈-alkyle ;
les cations d'imidazolium de formule générale (A) 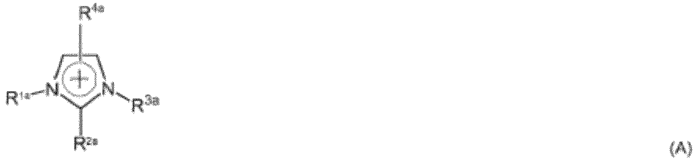
le noyau imidazole pouvant être substitué par au moins un groupe R^{na}, avec n = 1, 2, 3 ou 4, qui est choisi parmi les groupes C₁-C₈-alkyle, C₁-C₆-alcoxy, C₁-C₆-aminoalkyle substitué, C_{S}-C₁₂-aryle substitué ou (C₅-C₁₂-aryle substitué)-C₁-C₆-alkyle ;
les cations de pyridinium de formule générale (B) 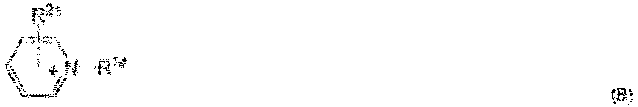
le noyau pyridine pouvant être substitué par au moins un groupe R^{na} avec n = 1 ou 2, qui est choisi parmi les groupes C₁-C₆-alkyle, C₁-C₆-alcoxy, C₁-C₆-aminoalkyle substitué, C_{S}-C₁₂-aryle substitué ou (C₅-C₁₂-aryle substitué)-C₁-C₆-alkyle ;
les cations de pyrazolium de formule générale (C) 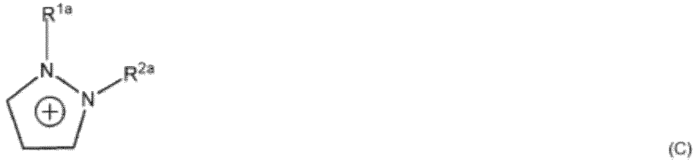
le noyau pyrazole pouvant être substitué par au moins un groupe R^{na} avec n = 1 ou 2, qui est choisi parmi les groupes C₁-C₆-alkyle, C₁-C₆-alcoxy, C₁-C₆-aminoalkyle substitué, C_{S}-C₁₂-aryle substitué ou (C₅-C₁₂-aryle substitué)-C₁-C₆-alkyle ;
les cations de triazolium de formule générale (D) 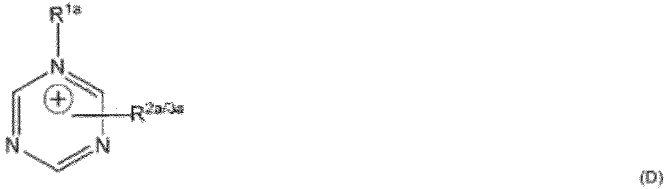
le noyau triazole pouvant être substitué par au moins un groupe R^{na} avec n = 1, 2 ou 3, qui est choisi parmi les groupes C₁-C₆-alkyle, C₁-C₆-alcoxy, C₁-C₆-aminoalkyle substitué, C_{S}-C₁₂-aryle substitué ou (C₅-C₁₂-aryle substitué)-C₁-C₆-alkyle.

9. Procédé selon la revendication 8, **caractérisé en ce qu'**un liquide ionique, choisi dans le groupe comprenant le bis(trifluorométhylsulfonyl)imide de 1-éthyl-3-méthylimidazolium ([EMIM][NTf₂]), l'hexafluorophosphate de 1-butyl-3-méthylimidazolium et le tétrafluoroborate de 1-butyl-3-méthylimidazolium, est utilisé.

10. Procédé selon l'une quelconque des revendications précédentes, **caractérisé en ce que** la composition dans l'étape b) présente une amine organique qui est différente du liquide ionique utilisé.

11. Procédé selon l'une quelconque des revendications précédentes, **caractérisé en ce que** la composition catalytique utilisée dans l'étape b) présente, comme matériau support, un oxyde d'aluminium, de silicium, de titane ou de zirconium ou du charbon actif ou des mélanges de ceux-ci.

12. Procédé selon l'une quelconque des revendications précédentes, **caractérisé en ce qu'**il s'agit, pour l'alcane, d'un ou de plusieurs Cₙ-alcanes (n = 3, 4 ou 5) ou de leurs mélanges et, pour l'alcène, de Cₙ-alcènes (n = 3, 4, 5) et de leurs mélanges.
